# EUROPEAN PATENT APPLICATION

(11) **EP 4 811 225 A1**
(43) Date of publication of application: **23.09.2026**
(21) Application number: 23958821.3
(22) Date of filing: 14.11.2023
(51) Int. Cl.: G06N 10/60, G16C 10/00

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: MATSUMOTO, Kazuhiro, Kawasaki-shi, Kanagawa 211-8588 (JP)
(74) Representative: Haseltine Lake Kempner LLP
(86) International application number: PCT/JP2023/040951
(87) International publication number: WO 2025/104812

(57) **Abstract**

An information processing device (100) calculates an excited state Sn(i) to be calculated based on a predetermined parameter value Pn. The information processing device (100) generates a candidate G(i) representing sets of mutually orthogonal states Sx(i) based on the calculated excited state Sn(i). The information processing device (100) calculates a set C(i) of energies Ex(i) for each state Sx(i) in each generated candidate G(i). The information processing device (100) assigns a rank R(i) for set C(i) of energies Ex(i) of calculated states Sx(i) in each candidate G(i). The information processing device (100) updates each state Sx(i) with one of the candidates G(i) based on the assigned rank R(i). The information processing device (100) changes the values of the predetermined parameter Pn so that the rank R(i) of the set C(i) of the energy Ex(i) of each updated state Sx(i) becomes higher in the coordinate system.

## Description

### TECHNICAL FIELD

The present invention relates to an information processing program, an information processing method, and an information processing device.

### BACKGROUND ART

In fields such as materials development and pharmaceutical development, computational chemistry is sometimes used to analyze the properties of candidate substances for materials or pharmaceuticals. When analyzing properties of a substance, it may be necessary to analyze not only a ground state but also, for example, an excited state so as to analyze properties such as whether the substance reacts or how readily a reaction proceeds. The ground state is the state with the lowest possible energy among possible energy states of a substance. The excited state is any state with higher energy than the ground state among the possible energy states of a substance. As a method for analyzing excited states of a substance along with the ground state thereof, a method called variational quantum calculation of excited states is known.

Prior arts include, for example, methods for extracting the optimal synthesis path from multiple synthesis paths for a target compound. Furthermore, there are techniques for calculating an expected value of a Hamiltonian by statistically processing the results of quantum computations performed for each initial state. Additionally, there are techniques for calculating the energy of the ground state of a molecular system using atomic coordinates and atomic charges of the system.

### [Prior Art Documents]

### [Patent Documents]

Patent Document 1: Japanese Laid-Open Patent Publication No. 2010-009257
Patent Document 2: International Publication No. WO 2020/090559
Patent Document 3: U.S. Patent Application Publication No. 2005/0273306

### DISCLOSURE OF INVENTION

### PROBLEM TO BE SOLVED BY THE INVENTION

However, in the prior arts, it is difficult to calculate the energy of an excited state of a material when analyzing the properties of the material. For example, in some instances the energy of an excited state of a material cannot be calculated together with the energy of the ground state thereof so as to satisfy a condition that the ground state and excited state of the material are orthogonal.

In one aspect, the present invention aims to calculate the energy of the excited state of a material.

### MEANS FOR SOLVING PROBLEM

According to an embodiment, the disclosed is an information processing program, an information processing method, and an information processing device including: when repeating by quantum chemical calculation, a specific computation for calculating each of a plurality of potential excited states of a substance, the specific computation being repeated based on a value of a parameter while changing the value of the parameter that is for expressing a target excited state to be calculated among the plurality of excited states, calculating based on the value of the parameter, the target excited state to be calculated; based on the calculated target excited state, for each of the plurality of potential excited states of the substance from a ground state of the substance to the target excited state to be calculated, fixing the each of the plurality of potential excited states of the substance and subtracting a projection component onto at least one state from another state so that the states are orthogonal to each other, thereby generating a plurality of candidates each representing a set of the states orthogonal to each other; calculating a set of energies of the states in each of the generated plurality of candidates; setting a rank for each calculated set of energies so that in a coordinate system containing axes representing the energies of each state, among a plurality of points respectively corresponding to the calculated sets of energies, a point relatively closer to an origin has a relatively higher rank; updating each state with one of the plurality of candidates, based on the set rank; and changing the value of the parameter so that in the coordinate system, the rank set for each set of energies of each updated state increases.

### EFFECT OF THE INVENTION

According to one aspect, it becomes possible to calculate the energy of an excited state of a substance.

### BRIEF DESCRIPTION OF DRAWINGS

Fig. 1 is an explanatory diagram depicting an example of an information processing method according to an embodiment.
Fig. 2 is an explanatory diagram depicting an example of an information processing system 200.
Fig. 3 is a block diagram depicting an example of a hardware configuration of an excitation energy calculating device 201.
Fig. 4 is an explanatory diagram depicting an example of energy calculation setting information 400.
Fig. 5 is a block diagram (Part 1) depicting an example of a functional configuration of the excitation energy calculating device 201.
Fig. 6 is a block diagram (Part 2) depicting an example of the functional configuration of the excitation energy calculating device 201.
Fig. 7 is a block diagram (Part 3) depicting an example of the functional configuration of the excitation energy calculating device 201.
Fig. 8 is an explanatory diagram depicting an example of stored contents of a first calculation result table 800.
Fig. 9 is an explanatory diagram depicting an example of stored contents of a second calculation result table 900.
Fig. 10 is an explanatory diagram depicting an example of generating candidates.
Fig. 11 is an explanatory diagram depicting an example of assigning a rank.
Fig. 12 is an explanatory diagram depicting an example of stored contents of the second calculation result table 900 after saving various information.
Fig. 13 is an explanatory diagram depicting an example of outputting calculation results.
Fig. 14 is a flowchart depicting an example of an overall processing procedure.
Fig. 15 is a flowchart depicting an example of a procedure of a first energy calculation process.
Fig. 16 is a flowchart (Part 1) depicting a procedure of a second energy calculation process.
Fig. 17 is a flowchart (Part 2) depicting a procedure of the second energy calculation process.
Fig. 18 is a flowchart depicting an example of an i-th energy calculation processing procedure.

### BEST MODE(S) FOR CARRYING OUT THE INVENTION

Embodiments of an information processing program, an information processing method, and an information processing device according to the present invention are described in detail with reference to the accompanying drawings.

### (Example of Information Processing Method According to Embodiment)

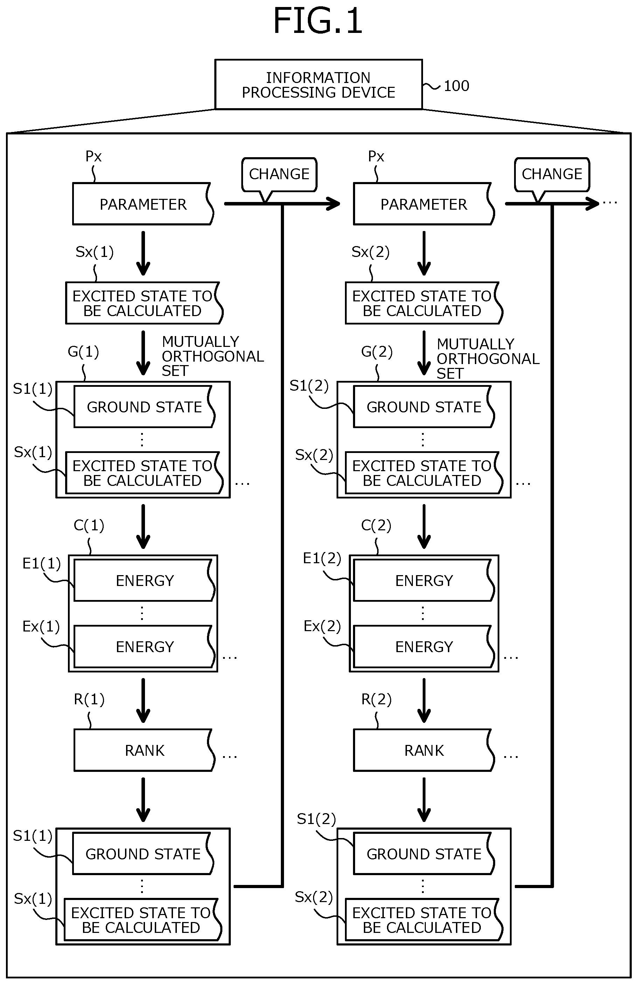

Fig. 1 is an explanatory diagram depicting an example of an information processing method according to an embodiment. In Fig. 1, the information processing device 100 is a computer for calculating the energy of an excited state of a substance. The information processing device 100 is, for example, a server or a PC (Personal Computer).

The substance is, for example, an atom or a molecule. The ground state is the state with the lowest energy among the possible energy states of the substance. An excited state is a state with higher energy than the ground state among the possible energy states of the substance.

For example, in fields such as materials development and pharmaceutical development, analyzing the ground state of a substance is important for analyzing the properties thereof. Furthermore, analyzing the excited state of a substance is necessary to analyze properties such as whether the substance reacts or how readily a reaction of the substance proceeds. Specifically, to analyze properties like a flame color reaction of a substance, it is necessary to analyze both the ground state and an excited state of the substance and calculate the energy difference between the ground state and the excited state.

Here, the various states including the ground state and the excited state of a substance are mutually orthogonal. Therefore, when analyzing one or more excited states of a substance and calculating the energy of each of these excited states, it is necessary to analyze the excited states under conditions that satisfy a constraint that the states of the substance are mutually orthogonal with each other. However, there is a problem in that it is difficult to analyze the excited states of the substance and calculate the energies of the excited states of the substance under conditions that satisfies the constraint that the states of the substance are mutually orthogonal with each other.

Conventionally, as a method for analyzing the excited states of a material along with ground state thereof, there is a method called Variational Quantum Calculation of Excited States. This method is an iterative solution technique that repeatedly calculates the energy of an excited state of a substance while changing parameters to analyze the excited states. This method analyzes the ground state and thereafter, analyzes one or more excited states, starting from the lowest energy state.

When analyzing any particular excited state using this method, the analysis of that particular excited state is performed so as to minimize the value of an objective function which represents the sum of the energy of that particular excited state and the degree of overlap between that excited state and other states. The objective function includes, for example, parameters related to the degree of overlap between states. The degree of overlap between states serves as an indicator of whether or not states are orthogonal to each other. A degree of overlap of zero indicates that the states are orthogonal with each other.

In this method, it may be difficult to analyze the excited states of a substance and calculate the energies thereof while satisfying the constraint that states of the substance are mutually orthogonal with each other. For example, even when the objective function value is minimized, the degree of overlap between states does not necessarily become zero, and the states do not necessarily become orthogonal. Therefore, the excited states cannot be analyzed with high precision. Furthermore, for instance, an upper limit may be set on the number of iterations in an iterative solution method, which can also prevent accurate analysis of the excited states.

Furthermore, when analyzing multiple excited states sequentially from the lowest energy state, when the preceding excited state cannot be analyzed accurately, the subsequent excited state cannot be analyzed accurately either. Specifically, when analyzing the subsequent excited state, the objective function includes the less accurate preceding excited state, preventing accurate analysis of the subsequent state. Furthermore, for example, the larger the increment by which parameters are changed at each step of an iterative solution method, the more difficult it tends to be to accurately analyze the excited state.

Therefore, in the present embodiment, an information processing method is described that can accurately calculate the energies of excited states of a substance such that the constraint that the states including the ground state and excited states of the substance are orthogonal with other is satisfied. Here, an example of processing by the information processing device 100 is described.

In Fig. 1, the information processing device 100 calculates the energy of the ground state of the substance using quantum chemistry calculations. Then, with respect to each of one or more excited states of the substance, starting from the lowest energy state, the information processing device 100 calculates the energy of each of the excited states. The substance is a substance requested for analysis. The substance may be, for example, candidates of a material or pharmaceutical.

The example in Fig. 1 explains how the information processing device 100 calculates the energy of an excited state for the one or more excited states of a substance. Each of the states including the ground state and one or more excited states of the substance, is possible energy state of the substance. The states are, for example, mutually independent. In other words, the states are, for example, mutually orthogonal with each other.

For example, the number of states for which energy is to be calculated, including the ground state, can be arbitrarily specified. In other words, the number of states for which the information processing device 100 calculates energies including the ground state, can be arbitrarily specified. Specifically, when the information processing device 100 calculates the energy for each of three states, including the ground state and two excited states, the number of states is specified as "3".

The information processing device 100 stores parameter values for representing each state, including the ground state and one or more excited states. The parameters for representing a state may be one or may be plural. For example, the parameters for representing a state may be angle parameters used in a quantum circuit representing that state. The angle parameters represent, for example, the angles of rotation around each axis such as the X-axis, Y-axis, or Z-axis. The range of the angle parameter for each axis may be set, for example, to 0 degrees or more but less than 360 degrees, respectively. Quantum chemistry calculations are a method for analyzing the structure or properties of atoms or molecules from the electronic states thereof. Quantum chemistry calculations are performed, for example, by a quantum computer or quantum simulation.

When calculating the energy of a certain excited state, the information processing device 100 calculates the certain excited state and calculates the energy thereof, by repeating a predetermined computation while changing the values of predetermined parameters representing the certain excited state. The predetermined computation is, for example, an operation that calculates the certain excited state based on the values of predetermined parameters using quantum chemical calculations.

In the following description, the state with the x-th lowest energy among the possible energy states of a substance may be assigned the symbol "Sx". Here, x is a natural number ranging from 1 to the specified number of states. For example, Sx (x=1) corresponds to the ground state. For example, Sx (x≥2) corresponds to an excited state. Furthermore, in the following description, the energy of state Sx may be denoted by the symbol "Ex".

The information processing device 100 specifically stores predetermined parameters Pn for representing an excited state under-calculation Sn. Specifically, the information processing device 100 calculates the excited state under-calculation Sn and calculates the energy En of the excited state under-calculation Sn, by repeatedly performing a series of processes corresponding to predetermined computations described in (1-1) to (1-6) below. Here, n is the number of specified states. In the following description, the state Sx in the predetermined computation performed at an i-th iteration may be denoted as "state Sx(i)". In the following description, the energy Ex of state Sx(i) may be denoted as "energy Ex(i)".

(1-1) The information processing device 100 calculates the excited state under-calculation Sn(i) based on the value of a predetermined parameter Pn.

(1-2) The information processing device 100 generates based on the calculated excited state under-calculation Sn(i), a candidate G(i) representing a set of states Sx(i) mutually orthogonal with each other. The states Sx(i) are, for example, respectively, the ground state S1(i) to the excited state under-calculation Sn(i). G(i) is a candidate representing a set of states Sx(i) subject to the predetermined computation performed at the i-th iteration. Multiple G(i) candidates exist. Specifically, the information processing device 100, for each state Sx(i), fixes the state Sx(i) and subtracts projection components onto at least one state from other states such that the states become orthogonal to each other and thereby generates candidate G(i).

(1-3) The information processing device 100 calculates a set C(i) of energies Ex(i) for each state Sx(i) in each generated candidate G(i). C(i) is the set of energies Ex(i) for each state Sx(i) in the predetermined computation performed at the i-th iteration.

(1-4) The information processing device 100 assigns (sets) a rank (priority) R(i) for each set C(i) of energies Ex(i) calculated for each state Sx(i) in each candidate G(i). The rank represents an evaluation of the set C(i). A higher rank indicates a higher evaluation. A high rank indicates a favorable rank. A high rank indicates a favorable evaluation of the rank. A low rank indicates an unfavorable rank. A low rank indicates an unfavorable evaluation of the rank. The rank may be an indicator value where, for example, a smaller numerical value indicates a higher evaluation. The rank may also be an indicator value where, for example, a larger numerical value indicates a higher evaluation.

Here, a rank indicates a higher rank (evaluation) when the numerical value is smaller. For example, a high rank indicates a smaller rank value. A low rank indicates a larger rank value. A high evaluation indicates a smaller rank value. A low evaluation indicates a larger rank value. However, a rank may also indicate a higher rank (evaluation) when the numerical value is larger.

The information processing device 100 sets the rank R(i) for the calculated set C(i) in each candidate G(i) such that, for example, in a coordinate system, the closer a point corresponding to the calculated set C(i) is to the origin, the higher is the rank thereof. The coordinate system includes axes representing the energy of each state. The coordinate system is an orthogonal coordinate system. The origin is the point where each axis intersects. Being closer to the origin indicates a smaller rank value. Being farther from the origin indicates a larger rank value. However, negative values may be calculated for the energy of each state. Therefore, it is preferable to set the origin not at a case where the energy is zero, but at a value smaller than all calculated energy values.

(1-5) Based on the set rank R(i), the information processing device 100 updates each state Sx(i) by one of the candidates G(i). For example, the information processing device 100 updates each state Sx(i) using the candidate G(i) with the highest rank R(i).

(1-6) The information processing device 100 changes the value of a predetermined parameter Pn so that the rank of the set C(i) of the energies Ex(i) of the updated states Sx(i) in the coordinate system becomes higher. The information processing device 100, for example, updates the value of the predetermined parameter Pn by searching within a preset range so that the rank of the set C(i) of the energies Ex(i) of updated states Sx(i) becomes higher in the next predetermined computation. The search is, for example, realized by an optimization algorithm.

Thus, the information processing device 100 can perform the predetermined computation, calculate the excited state under-calculation Sn such that each state Sx is orthogonal, and calculate the energy En of the excited state under-calculation Sn. By repeating the predetermined computation, the information processing device 100 can accurately calculate the excited state under-calculation Sn and accurately calculate the energy En of the excited state under-calculation Sn while satisfying the constraint that the states Sx are orthogonal.

For example, the number of specified states is set to "2", and the states are designated as "state S1, S2". State S1 corresponds to the ground state. State S2 corresponds to the next lowest energy excited state next to the ground state. In the following description, an x-th excited state from the lowest energy may be denoted as the "x-th excited state". Therefore, state S2 corresponds to the first excited state.

The information processing device 100 specifically stores an initial value for parameter P1 representing the ground state S1. The information processing device 100 specifically stores an initial value for a first parameter P2 representing the first excited state S2.

The information processing device 100 calculates the ground state S1 based on the initial value of parameter P1 for representing the ground state S1, and calculates the energy E1 of the ground state S1. The information processing device 100 calculates the ground state S1 and the energy E1 of the ground state S1 using existing techniques such as VQE (Variational Quantum Eigensolver). Specifically, the information processing device 100 calculates the ground state S1 and the energy E1 of the ground state S1 by repeatedly performing the operation of calculating the ground state S1 with using a quantum chemical calculation while changing the value of the parameter P1 from the initial value.

While the description pertains to the information processing device 100 calculating the ground state S1 and the energy E1 of the ground state S1, configuration is not limited hereto. For example, the information processing device 100 may accept setting of an initial value for the ground state S1 and an initial value for the energy E1 of the ground state S1. In this case, the information processing device 100 needs not calculate the ground state S1 nor the energy E1 of the ground state S1.

The information processing device 100 sets the first excited state S2 as the excited state whose energy is to be calculated. The information processing device 100 calculates the first excited state S2 by repeating the first computation while changing the value of the first parameter P2 representing the first excited state S2, and calculates the energy E2 of the first excited state S2. The first computation, for example, calculates the first excited state S2 based on the value of the first parameter P2 using quantum chemical calculations.

First, the information processing device 100 performs a first execution of the first computation. The information processing device 100 calculates a first excited state S2(1), for example, based on the value of the first parameter P2.

The information processing device 100 generates a candidate G(1) representing a set of mutually orthogonal states Sx(1), for example, based on a calculated first excited state S2(1). Specifically, the information processing device 100, with respect to each state Sx(1), fixes the state Sx(1) and subtracts projection components onto at least one state from other states such that the states are orthogonal to each other and thereby generates candidate G(1).

The information processing device 100 calculates, for example, a set C(1) of energies Ex(1) for each state Sx(1) of each generated candidate G(1). The information processing device 100 assigns a rank R(1) for each set C(1) such that the rank thereof is higher the closer that the point corresponding to the set C(1) is to the origin in a first coordinate system. The first coordinate system includes axes representing the energy Ex(1) of each state Sx(1).

The information processing device 100 updates each state Sx(1) with one of the candidates G(1), for example, based on the set rank R(1). The information processing device 100, for example, updates the energy Ex(1) of each state Sx(1) with the set C(1) that corresponds to the candidate G(1).

The information processing device 100, for example, updates the value of the first parameter P2 such that the rank of the set C(1) of the energies Ex(1) of each updated state Sx(1) becomes higher in the first coordinate system. Specifically, the information processing device 100 updates the value of the first parameter P2 by searching within a predetermined range such that the rank of the set C(1) corresponding to the energy Ex(1) of each updated state Sx(1) becomes higher in the next execution of the first computation. The search is realized, for example, by an optimization algorithm.

The information processing device 100 performs second and subsequent executions of the first computation in the same manner until the first computation is performed a predetermined number of times. The predetermined number of times can be set arbitrarily. The predetermined number of times is the upper limit on the number of times the first computation is repeated. After executing the first computation a predetermined number of times, the information processing device 100 outputs the energy Ex of each last updated state Sx. For example, after executing the first computation a predetermined number of times, the information processing device 100 may associated and output the last updated states Sx and the respective last updated energies Ex thereof.

This enables the information processing device 100 to repeatedly update each state Sx in an appropriate direction and to repeatedly update the energy Ex of each state Sx in an appropriate direction. The information processing device 100 can accurately determine each state Sx and accurately determine the energy Ex of each state Sx, satisfying the constraint that the states are orthogonal to each other.

The information processing device 100 can, for example, vary parameter values to increase the ranks thereof and optimize each state Sx so that the energy Ex thereof is reduced overall. Consequently, the information processing device 100 can, for example, efficiently and accurately determine the energy of the excited state under-calculation Sn.

The information processing device 100 can, for example, adjust the previously calculated state Sx (x<n) along with the excited state under-calculation Sn when calculating the energy En of the excited state under-calculation Sn, thereby adjusting the energy Ex(x<n) of the state Sx (x<n). The information processing device 100 can, for example, via an upper limit of the number of iterations of a predetermined computation, improve the accuracy of the previously calculated state Sx (x<n) along with the excited state under-calculation Sn, even when the accuracy of the previously calculated state Sx (x<n) is poor. Therefore, the information processing device 100 can, for example, accurately determine each state Sx and accurately determine the energy Ex thereof.

Here, while the functions of the information processing device 100 have been described as being implemented by a single computer, configuration is not limited hereto. For example, the functions of the information processing device 100 may be implemented through the collaboration of multiple computers. For instance, the functions of the information processing device 100 may be implemented by multiple computers in a cloud environment.

### (Example of Information Processing System 200)

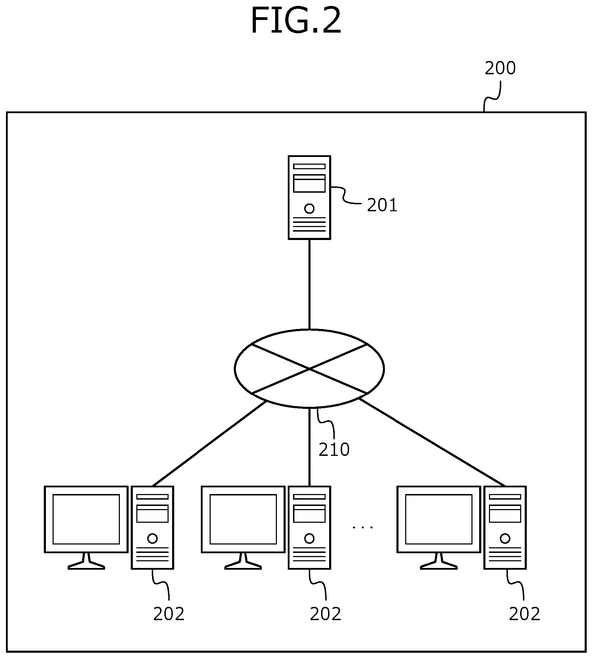

Next, with reference to Fig. 2, an example of an information processing system 200 to which the information processing device 100 depicted in Fig. 1 is applied, will be described. Here, an example is described where the information processing device 100 depicted in Fig. 1 is applied to the excitation energy calculating device 201 in the information processing system 200.

Fig. 2 is an explanatory diagram depicting an example of the information processing system 200. In Fig. 2, the information processing system 200 includes an excitation energy calculating device 201 and client devices 202.

In the information processing system 200, the excitation energy calculating device 201 and the client device 202 are coupled via a wired or wireless network 210. The network 210 may be, for example, a LAN (Local Area Network), WAN (Wide Area Network), or the Internet.

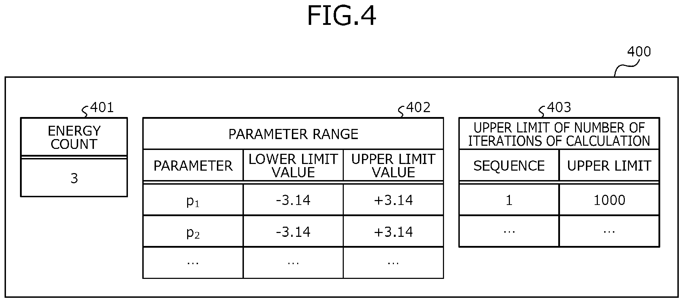

The excitation energy calculating device 201 is a computer that calculates the energy of an excited state of a substance in addition to the ground state thereof. The excitation energy calculating device 201 receives a processing request from the client device 202, requesting the calculation of the energy of an excited state of a substance-under-analysis. The processing request may include, for example, information identifying the substance-under-analysis. The processing request may also include, for example, energy calculation setting information 400, which will be described later with reference to Fig. 4. The energy calculation setting information 400 includes various settings related to energy calculation.

The various settings specify, for example, the range of energies to be calculated from the ground state energy of the substance-under-analysis to the energies of any excited states of the substance-under-analysis. Specifically, the various settings define the number of states of the substance-under-analysis, for which energy is to be calculated. The various settings also define, for example, how the iterative solution method for calculating the energy of each state of the substance-under-analysis is to be implemented. Specifically, the various settings define, for example, the number of iterations for the energy calculation which is a step in the iterative solution method. An example of the energy calculation setting information 400 will be described later with reference to Fig. 4.

The excitation energy calculating device 201 calculates, in response to a processing request, the energy of the ground state of the substance-under-analysis and the energy of at least one of the excitation states of the substance-under-analysis. The excitation energy calculating device 201 then transmits the calculated energy of at least one excited state of the substance-under-analysis to the client device 202. The excitation energy calculating device 201 may also transmit the calculated ground state energy of the substance-under-analysis to the client device 202. The excitation energy calculating device 201 may be, for example, a server or a PC.

The client device 202 is a computer used by a user. The user is, for example, an analyst who analyzes properties of the substance-under-analysis. Based on operational input of the analyst, the client device 202 generates a processing request requesting calculation of the energy of an excited state of the substance-under-analysis and transmits it to the excitation energy calculating device 201.

The client device 202 receives the energy of at least one excited state of the substance-under-analysis from the excitation energy calculating device 201. The client device 202 outputs the energy of at least one excited state of the substance-under-analysis for the analyst to reference.

The client device 202 may receive the ground state energy of the substance-under-analysis from the excitation energy calculating device 201. The client device 202 may output the ground state energy of the substance-under-analysis for the analyst to reference. The client device 202 may be, for example, a PC, a tablet device, or a smartphone.

The description here pertains to a case where the excitation energy calculating device 201 is a computer different from the client device 202, but this is not limited thereto. For example, the excitation energy calculating device 201 may have the functionality of a client device 202 and may also operate as a client device 202.

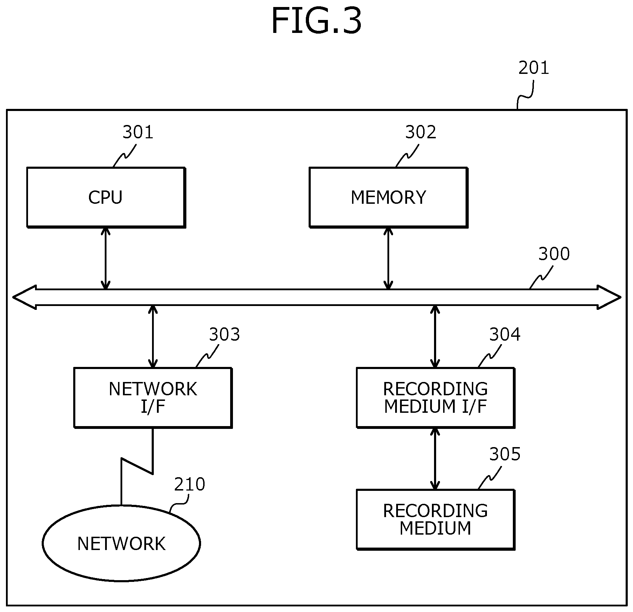

(Example of Hardware Configuration of Excitation Energy Calculating Device 201) Next, an example of a hardware configuration of the excitation energy calculating device 201 is described with reference to Fig. 3.

Fig. 3 is a block diagram of an example of a hardware configuration of the excitation energy calculating device 201. In Fig. 3, of the excitation energy calculating device 201 has a central processing unit (CPU) 301, a memory 302, a network interface (I/F) 303, a recording medium I/F 304, and a recording medium 305. Further, the components are connected to each other by a bus 300.

Here, the CPU 301 governs overall control of the information processing device 100. The memory 302, for example, includes a read-only memory (ROM), a random access memory (RAM), and a flash-ROM. In particular, for example, the flash-ROM and/or ROM stores therein various programs and the RAM is used as a work area of the CPU 301. Programs stored to the memory 302 are loaded onto the CPU 301, whereby encoded processes are executed by the CPU 301.

The network I/F 303 is coupled to the network 210 via a communications line and is coupled to other computers through the network 210. Further, the network I/F 303 administers an internal interface with the network 210 and controls the input and output of data with respect to the other computers. The network I/F 303, for example, is a modem, a LAN adapter, or the like.

The recording medium I/F 304 controls the reading and writing of data with respect to the recording medium 305 under the control of the CPU 301. The recording medium I/F 304 is, for example, a disk drive, a solid-state drive (SSD), a universal serial bus (USB) port, or the like. The recording medium 305 is a nonvolatile memory storing data written thereto under the control of the recording medium I/F 304. The recording medium 305 is, for example, a disk, a semiconductor memory, a USB memory, or the like. The recording medium 305 may be removable from the excitation energy calculating device 201.

In addition to the components above, the excitation energy calculating device 201 may include, for example, a keyboard, a mouse, a display, a printer, a scanner, a microphone, a speaker, etc. Further, the excitation energy calculating device 201 may further have the recording medium I/F 304 and/or the recording medium 305 in plural. The information processing device 100 may omit the recording medium I/F 304 and/or the recording medium 305.

### (Example of Hardware Configuration of Client Device 202)

An example of a hardware configuration of each client device 202 is basically a same as the example of the hardware configuration example of the excitation energy calculating device 201 depicted in Fig. 3 and thus, the description is omitted. However, the client devices 202 may include, in addition to the components depicted in Fig. 3, for example, a keyboard, a mouse, and a display.

### (Example of Energy Calculation Setting Information 400)

Next, an example of energy calculation setting information 400 is described with reference to Fig. 4.

Fig. 4 is an explanatory diagram depicting an example of energy calculation setting information. In Fig. 4, the energy calculation setting information 400 includes a number of energies 401, parameter ranges 402, and an upper limit 403 of the number of iterations of calculation.

The number of energies 401 indicates the number of energies to be calculated. The number of energies 401 corresponds to the number of states of the substance. For example, when the number of energies 401 is "3", the energies of the ground state, the first excited state, and the second excited state are calculated.

The parameter ranges 402 indicates the ranges of the parameters used to represent respective states of the substance-under-analysis. The parameters are, for example, p₁, p₂. The parameter ranges 402 include the lower limit and upper limit values for each parameter. For example, parameter ranges 402 include a lower limit value "-3.14" and an upper limit value "+3.14" for parameter p₁. Parameter ranges 402 also include a lower limit value "-3.14" and an upper limit value "+3.14" for parameter p₂.

The upper limit of the number of iterations of calculation indicates the upper limit of the number of iterations for performing energy calculations for each state of the substance-under-analysis. Specifically, the upper limit of the number of iterations of calculation indicates a sequence specifying a state of the substance-under-analysis and a corresponding upper limit for the number of iterations for performing energy calculations for the state. The sequence indicates the position of the state among the possible energy states of the substance-under-analysis, starting from the state with the lowest energy.

For example, for the ground state, which is the first state in order of increasing energy among the possible energy states of the substance-under-analysis, the upper limit for the number of iterations for performing energy calculations is "1000". In this case, for the ground state, the energy calculation of the iterative solution method is performed 1000 times while changing the parameter values. The energy calculation is the process of calculating the energy of one of the states of the substance-under-analysis.

### (Functional Configuration Example of Excitation Energy Calculating Device 201)

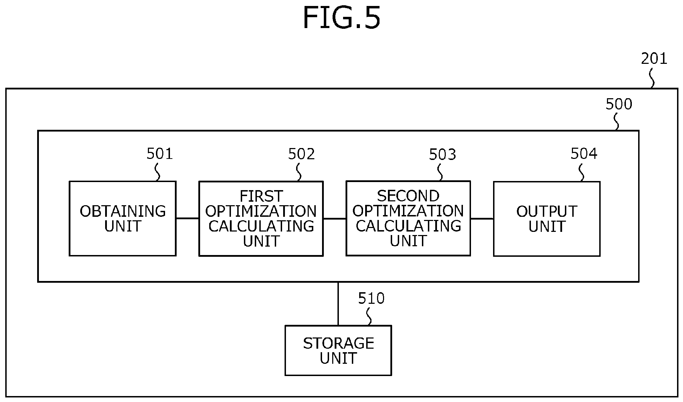

Next, with reference to Figs. 5 to 7, an example of a functional configuration of the excitation energy calculating device 201 will be described.

Figs. 5, 6, and 7 are block diagrams depicting an example of the functional configuration of the excitation energy calculating device 201. In Fig. 5, the excitation energy calculating device 201 includes an obtaining unit 501, a first optimization calculating unit 502, a second optimization calculating unit 503, an output unit 504, and a storage unit 510.

The storage unit 510 is implemented, for example, by a storage area such as the memory 302 or recording medium 305 depicted in Fig. 3. Herein, while a case in which the storage unit 510 is included in the excitation energy calculating device 201, configuration is not limited hereto. For example, the storage unit 510 may be included in a device apart from the excitation energy calculating device 201, and the contents stored in the storage unit 510 may be accessible from the excitation energy calculating device 201.

The obtaining unit 501 to output unit 504 function as an example of controller 500. Specifically, functions of the obtaining unit 501 to output unit 504 are implemented, for example, by executing on the CPU 301, a program stored in a storage area such as memory 302 or the recording medium 305 depicted in Fig. 3, or via the network I/F 303. The results of processing by the functional units are stored to a storage area, such as the memory 302 or the recording medium 305 depicted in Fig. 3.

The storage unit 510 stores various types of information referenced or updated in the processes by the functional units. The storage unit 510 stores, for example, the number Ns of states each of whose energy is to be calculated. The storage unit 510 stores, for example, the range of parameters for representing each state. The storage unit 510 stores, for example, the upper limit of the number of iterations for the energy calculation used to calculate the energy of each state. Specifically, the memory unit 510 stores the energy calculation setting information 400. The energy calculation setting information 400 is obtained, for example, by the obtaining unit 501. The energy calculation setting information 400 may be stored in advance, for example.

The storage unit 510 stores, for example, parameter values for representing each state. The parameter values for representing each state are, for example, obtained by the obtaining unit 501 and updated by the first optimization calculating unit 502 or the second optimization calculating unit 503. The storage unit 510 stores, for example, each state. Each state is calculated, for example, by the first optimization calculating unit 502 or the second optimization calculating unit 503. The storage unit 510 stores, for example, the energy of each state. The energy of each state is calculated, for example, by the first optimization calculating unit 502 or the second optimization calculating unit 503.

Obtaining unit 501 obtains various information used for processing by each functional unit. Obtaining unit 501 stores the obtained various information in storage unit 510 or outputs it to each functional unit. Obtaining unit 501 may also output various information stored in storage unit 510 to each functional unit. Obtaining unit 501 obtains various information based on, for example, user manipulations input. Obtaining unit 501 may, for example, receive various information from a device different from the excitation energy calculating device 201.

The obtaining unit 501 obtains, for example, a processing request requesting calculation of the energy of an excited state of a substance-under-analysis. Specifically, the obtaining unit 501 obtains the processing request by receiving the processing request from another computer. The other computer is, for example, the client device 202 depicted in Fig. 2. Furthermore, the obtaining unit 501 may specifically obtain the processing request by accepting input of the processing request based on user operational input.

The obtaining unit 501 obtains, for example, information identifying the substance-under-analysis. Specifically, the obtaining unit 501 obtains the information identifying the substance-under-analysis by receiving the information from another computer. The other computer is, for example, the client device 202 depicted in Fig. 2. Furthermore, obtaining unit 501 may obtain information identifying the substance-under-analysis by accepting input of such information based on user operational input. Additionally, obtaining unit 501 may obtain information identifying the substance-under-analysis by extracting the information from the processing request when such information is included therein.

The obtaining unit 501 obtains, for example, the energy calculation setting information 400. Here, the energy calculation setting information 400 includes various settings related to energy calculations for computing each state of the substance-under-analysis. The energy calculation setting information 400 includes, for example, the number Ns of states for which energy is to be calculated. The energy calculation setting information 400 includes, for example, a range of parameters representing each state of the substance-under-analysis. The energy calculation setting information 400 includes, for example, the upper limit of the number of iterations for the energy calculation used to calculate the energy of each state of the substance-under-analysis.

The obtaining unit 501 specifically obtains the energy calculation setting information 400 depicted in Fig. 4 by receiving the energy calculation setting information 400 from another computer. The other computer is, for example, a client device 202. Furthermore, the obtaining unit 501 may obtain the energy calculation setting information 400 by accepting input of the energy calculation setting information 400 based on user operational input. Furthermore, the obtaining unit 501 may obtain the energy calculation setting information 400 by extracting the energy calculation setting information 400 from a processing request when the energy calculation setting information 400 is included in the processing request.

The obtaining unit 501 obtains, for example, the number Ns of states whose energies are to be calculated, based on the energy calculation setting information 400. The obtaining unit 501 obtains, for example, the range of parameters for representing each state, based on the energy calculation setting information 400. The obtaining unit 501 obtains, for example, the upper limit of the number of iterations for the energy calculation to calculate the energy of each state, based on the energy calculation setting information 400.

The obtaining unit 501 may, for example, obtain initial values for parameters representing each state. The obtaining unit 501 may, for example, obtain initial values for each state. The obtaining unit 501 may, for example, obtain initial values for the energy of each state. The obtaining unit 501 may, for example, obtain initial values for the rankings of each set of initial energy values for each state.

The obtaining unit 501 may receive a start trigger to initiate the process of any functional unit. The start trigger may be, for example, a predetermined manipulation input by a user. The start trigger may be, for example, the reception of predetermined information from another computer. The start trigger may, for example, be the output of predetermined information by any functional unit. Specifically, the obtaining unit 501 may regarding obtaining a processing request as the start trigger for initiating the processes of first optimization calculating unit 502 and the second optimization calculating unit 503.

The first optimization calculating unit 502 repeats a predetermined energy calculation to compute the ground state based on the parameter values, using quantum chemical calculations while varying the parameter values representing the ground state. By repeating the predetermined energy calculation, the first optimization calculating unit 502 computes the ground state and calculates the energy of the ground state.

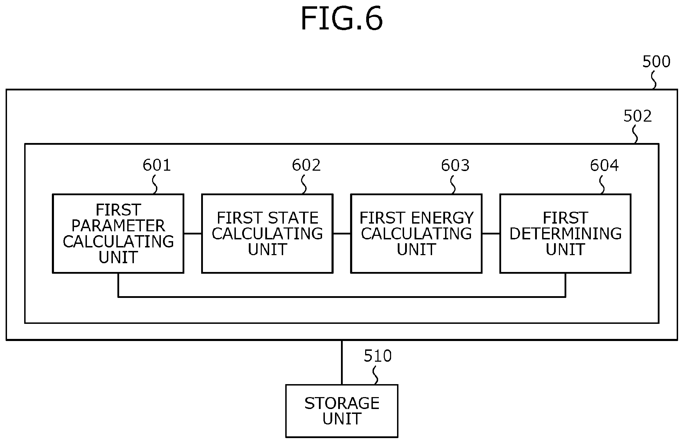

In the following description, j-th state from the lowest energy among possible energy states of the substance-under-analysis may be denoted as "state Stateⱼ". j is 1, 2, ..., Ns. State₁ corresponds to the ground state. State Stateⱼ (j≥2) corresponds to an excited state. Furthermore, in the following description, the energy of state Stateⱼ may be denoted as "energy Energyⱼ". Now, moving to the description of Fig. 6, details of the first optimization calculating unit 502 will be explained.

In Fig. 6, the first optimization calculating unit 502 includes a first parameter calculating unit 601, a first state calculating unit 602, a first energy calculating unit 603, and a first determining unit 604.

The first parameter calculating unit 601 sets the values {p₁, p₂, ..., p_{Np}} of the parameter P₁ for representing the ground state State₁. Np is the number of parameter values. For example, in the first energy calculation, the first parameter calculating unit 601 sets the values of the parameter P₁ representing the ground state State₁ to initial values. The initial values may be selected randomly, for example. This allows the first parameter calculating unit 601 to set the values of parameter P₁ representing the ground state State₁ and enables the execution of the energy calculation.

For subsequent energy calculations, such as the second and later ones, the first parameter calculating unit 601 changes the values of parameter P₁ so that the energy Energy₁ of the ground state State₁ becomes lower. Specifically, the first parameter calculating unit 601 changes the values of parameter P₁ by searching for values of parameter P₁ within a preset range that lowers the energy Energy₁ of the ground state State₁. The search is performed, for example, using an optimization algorithm. This enables the first parameter calculating unit 601 to appropriately vary the values of parameter P₁ representing the ground state State₁, thereby enabling the ground state State₁ to be calculated accurately.

The first state calculating unit 602 calculates the ground state State₁ based on the values of parameter P₁, using quantum chemical calculations. The first state calculating unit 602, for example, by executing quantum chemical calculations using a quantum computer or quantum simulation, sets the values of parameter P₁ in the quantum circuit representing the ground state State₁and calculates the ground state State₁. The quantum computer may be incorporated in the excitation energy calculating device 201, or may be an external device coupled to the excitation energy calculating device 201 via the network 210 depicted in Fig. 2. Specifically, the first state calculating unit 602 calculates the ground state State₁ according to the following equation (1).${State}_{j} \left(m\right) = {\sum }_{n = 1}^{{N}_{v}} {CalclateState}_{{p}_{1} , {p}_{2} , ⋯ , {p}_{{N}_{p}}} \left(m, n\right) \times {Tentative}_{j} \left(n\right)$

Here, j is 1. Let Nv=2^{Ns}. A state Stateⱼ(m) is represented, for example, by a complex number. A complex number has a real part and an imaginary part. Therefore, for each state Stateⱼ(m), two elements (the real part and the imaginary part) exist. Nv corresponds to the number of all combinations that should be calculated as state Stateⱼ. Stateⱼ(m) is an array with m components. Furthermore, Tentativeⱼ(n) is a tentative vector of a complex number representing state Stateⱼ. Tentativeⱼ(n) is an array with n components.

Furthermore, CalculateState_{p1, p2,}... _{, pNp} is a function including the parameter values p₁, p₂, ..., p_{Np}. CalculateState_{p1, p2,}..._{, pNp} is, for example, an m-row × n-column matrix determined by the parameter values p₁, p₂, ..., p_{Np}. Furthermore, m is 1, 2, ..., Nv. n is 1, 2, ..., Nv. Thus, the first state calculating unit 602 can calculate the ground state State₁.

For Tentativeₖ(l), the complex conjugate TentativeComplexConjugateₖ(l) is defined by the following equation (2). k is 1, 2, ..., Ns. l is 1, 2, ..., Nv.$\begin{matrix}{TentativeComplexConjugate}_{k} \left(l\right) = real _ part \left({Tentative}_{k}\left(l\right)\right) - i \times \\ imaginary _ part \left({Tentative}_{k}\left(l\right)\right)\end{matrix}$

The first energy calculating unit 603 calculates the energy Energy₁ of the calculated ground state State₁. The first energy calculating unit 603 calculates the energy Energy₁ of the calculated ground state State₁, for example, according to the following equation (3). ${Energy}_{j} = {\sum }_{l = 1}^{{N}_{v}} {\sum }_{m = 1}^{{N}_{v}} \begin{matrix}{StateComplexConjugate}_{j} \left(l\right) \\ \times CalclateEnergy \left(l, m\right) \\ \times {State}_{j} \left(m\right)\end{matrix}$

Here, for Stateⱼ(l), the complex conjugate StateComplexConjugateⱼ(l), which has the real part unchanged and the sign of the imaginary part inverted, is defined by the following equation (4). StateComplexConjugateⱼ(l) is an array with l (L) components. Furthermore, CalculateEnergy(l, m) is an l (L)-row × m-column matrix.$\begin{matrix}{StateComplexConjugate}_{j} \left(l\right) = real _ part \left({State}_{j}\left(l\right)\right) - i \times \\ imaginary _ part \left({State}_{j}\left(l\right)\right)\end{matrix}$

The first determining unit 604 determines whether the number of iterations of the energy calculation has exceeded the upper limit corresponding to the ground state State₁. When the number of energy calculation iterations has not exceeded the upper limit corresponding to the ground state State₁, the first determining unit 604 controls the functional units such that the first parameter calculating unit 601 to the first energy calculating unit 603 execute the energy calculation again. When the number of energy calculation iterations exceeds the upper limit corresponding to the ground state State₁, the first determining unit 604 terminates the energy calculation iteration.

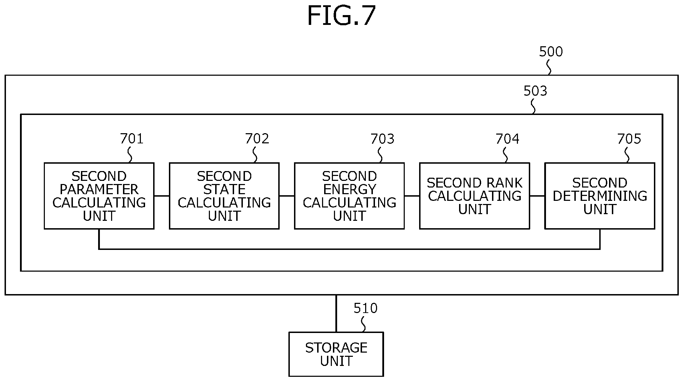

Here, reference of the description returns to Fig. 5; the second optimization calculating unit 503 targets each of one or more excited states of the substance in ascending order of energy and calculates the energy of the excited state to be calculated. Based on the values of parameters representing the excited state that is to be calculated, the second optimization calculating unit 503 repeats a specific energy calculation to compute the excited state that is to be calculated, while the second optimization calculating unit 503 varies the parameter values. The second optimization calculating unit 503 calculates the excited state that is to be calculated by repeating the specific energy calculation, and calculates the energy of the excited state that is to be calculated. Now, moving to the description of Fig. 7, the contents of the second optimization calculating unit 503 will be explained.

In Fig. 7, the second optimization calculating unit 503 includes a second parameter calculating unit 701, a second state calculating unit 702, a second energy calculating unit 703, a second ranking calculating unit 704, and a second determining unit 705.

The second parameter calculating unit 701 sets the values {p₁, p₂, ..., p_{Np}} of the parameter Pₓ representing the excitation state Stateₓ that is to be calculated. Here, 2≤x≤Ns is satisfied. For example, in the first energy calculation, the second parameter calculating unit 701 initializes the values of the parameter Pₓ representing the excited state Stateₓ that is to be calculated. The initial values may be selected randomly, for example. This enables the second parameter calculating unit 701 to set the values of the parameter Pₓ representing the excited state Stateₓ that is to be calculated and perform the energy calculation.

For energy calculations after the second execution thereof, the second parameter calculating unit 701 changes the values of parameter Pₓ such that the rank of the energy set for each state State₁ to Stateₓ updated in the current energy calculation becomes higher in a predetermined coordinate system. Each state ranges from the ground state State₁ to the excited state Stateₓ that is to be calculated. The predetermined coordinate system includes, for example, axes representing the energy of each state State₁ to Stateₓ.

The second parameter calculating unit 701 specifically searches for values of parameter Pₓ that will result in a higher ranking of the set (group) of energies Energy₁ to Energyₓ of the states State₁ to Stateₓ which are updated by the energy calculation of this time. The search is performed, for example, using an optimization algorithm. Specifically, the second parameter calculating unit 701 changes the values of parameter Pₓ by searching for the values within a preset range. This enables the second parameter calculating unit 701 to appropriately vary the values of parameter Pₓ representing the excitation state Stateₓ that is to be calculated, thereby enabling accurate calculation of the excitation state Stateₓ that is to be calculated.

The second state calculating unit 702 calculates, by quantum chemical calculation, the excited state Stateₓ that is to be calculated, based on the values of the parameter Pₓ for representing the excited state Stateₓ that is to be calculated. The second state calculating unit 702, for example, by executing quantum chemical calculations using a quantum computer or quantum simulation, sets the values of a parameter eₓ into the quantum circuit representing the excited state Stateₓ that is to be calculated, and calculates the excited state Stateₓ that is to be calculated. The quantum computer may be incorporated in the excitation energy calculating device 201, or may be an external device connected to the excitation energy calculating device 201 via the network 210 as depicted in Fig. 2. The second state calculating unit 702 specifically calculates the excited state Stateₓ that is to be calculated, according to the above equation (1). This enables the second state calculating unit 702 to calculate the excited state Stateₓ that is to be calculated, and to bring the excited state Stateₓ that is to be calculated, closer to the desired direction.

Based on the calculated excitation state Stateₓ, the second state calculating unit 702 generates candidates representing sets of mutually orthogonal states State₁ to State_{x,} with respect to states State₁ to Stateₓ. The second state calculating unit 702 generates one or more candidates, for example, by fixing state Stateₖ and subtracting the projection component projected onto at least one state from another state Stateⱼ (j≠k) such that the states become orthogonal with each other. Specifically, the second state calculating unit 702 fixes state Stateₖ and normalizes state Stateⱼ (j≠k) by subtracting the projection component onto at least one state from Stateⱼ, according to Equations (5) to (7) below. Thus, the second state calculating unit 702 can generate candidates representing sets of states State₁ to Stateₓ such that the constraint that the states are orthogonal is satisfied.${State}_{after _ projection , k} \left(l\right) = {State}_{before _ projection , k} \left(l\right)$$\begin{matrix}{State}_{after _ projection , j} \left(l\right) \\ = {State}_{before _ projection , j} \left(l\right) \\ - {State}_{before _ projection , k} \left(l\right) \\ \times {\sum }_{m = 1}^{{N}_{v}} {StateComplexConjugate}_{before _ projection , k} \left(m\right) \\ \times {State}_{before _ projection , j} \left(m\right)\end{matrix}$${State}_{after _ normalization , j} \left(l\right) = \frac{{State}_{before _ normalization , j} \left(l\right)}{\sqrt{\begin{matrix}{\sum }_{m = 1}^{{N}_{v}} {StateComplexConjugate}_{before _ normalization , j} \left(m\right) \\ \times {State}_{before _ normalization , j} \left(m\right)\end{matrix}}}$

Here, State_{after_projection,k}(l) is the fixed state Stateₖ. State_{after_projection,k}(l) is an array with I (L) components. State_{before_projection,k}(l) is the state Stateₖ before being fixed. State_{before_projection,k}(l) is an array **with** I **(L) components.**

State_{after_projection,j}(l) is Stateⱼ after subtracting the projection component. State_{after_projection,j}(l) is an array with I (L) components. State_{before_projection,j}(m) is the state Stateⱼ before subtracting the projection components. State_{before_projection,j}(m) is an array with m components. StateComplexConjugate_{before_projection,j}(m) is the complex conjugate of State_{before_projection,j}(m). StateComplexConjugate_{before_projection,j}(m) is an array with m components.

State_{after_normalization,j}(l) is the normalized Stateⱼ. State_{after_normalization,j}(l) is an array with I (L) components. State_{before_normalization,j}(I) is the state Stateⱼ before normalization.State_{before_normalization,j}(l) is an array with I (L) components. State_{before_normalization,j}(l) is, for example, set to State_{after_projection,j}(l).

State_{before_normalization,j}(m) is the Stateⱼ before normalization. State_{before_normalization,j}(m) is an array with m components. State_{before_normalization,j}(m) is, for example, set to State_{after_projection,j}(m). StateComplexConjugate_{before_normalization,j}(m) is the complex conjugate of State_{before_normalization,j}(m). StateComplexConjugate_{before_normalization,j}(m) is an array with m components.

The second energy calculating unit 703 calculates sets of energies Energy, to Energyₓ for each state State₁ to Stateₓ in each generated candidate. For each generated candidate, the second energy calculating unit 703 calculates, for example, according to the above equation (3), a set of energies Energy₁ to Energyₓ for each state State₁ to Stateₓ in each generated candidate₁. This allows the second energy calculating unit 703 to obtain a criterion for determining which candidate is preferable.

The second ranking calculating unit 704 sets a rank for each set of energies Energy₁ to Energyₓ of each calculated state. The second ranking calculating unit 704 sets the ranking for the set of energies Energy₁ to Energyₓ of each candidate such that, for example, in a coordinate system, a point corresponding to a set of energies Energy₁ to Energyₓ relatively closer to the origin has a relatively higher ranking.

As for the energy Energyⱼ of each state Stateⱼ, a negative value may be calculated. Therefore, it is preferable to set the origin not at the case where the energy is 0, but at a value smaller than all calculated energy values. Furthermore, a smaller numerical value indicates a higher rank (evaluation).

The proximity between any point corresponding to the sets of energies Energy₁ to Energyₓ and the origin is defined, for example, by the Euclidean distance between the point and the origin. The proximity between any point corresponding to the sets of energies Energy₁ to Energyₓ and the origin may be defined, for example, by the number of other points therebetween all of which correspond to energy Energy₁ to Energyₓ lower than energy corresponding to said any point. This allows the second ranking calculating unit 704 to obtain a criterion for determining which candidate is preferable.

Specifically, in the coordinate system, it is possible that for any specific set of calculated energies Energy₁ to Energyₓ, another set exists with the same distance from the origin. In this case, the second rank calculating unit 704 selects an energy Energyᵢ from among Energy₁ to Energyₓ. The energy Energyᵢ is one that first differs between the specific set and the other set, starting from the lower energy. Specifically, the second rank calculating unit 704 assigns a higher rank to the specific set than the other set when the selected energy Energyᵢ is lower than that of the other set. Specifically, the second rank calculating unit 704 assigns a lower rank to the specific set than the other set when the selected energy Energyᵢ is higher than that of the other set. For example, in reality, the state of a substance tends to be the state with the lowest possible energy. The second rank calculating unit 704 can set an appropriate rank for any specific set based on the relatively low energy Energyᵢ, while taking this tendency into account.

More specifically, the second rank calculating unit 704 assigns a higher rank to any specific set when the energy Energy₁ thereof is lower than that of another set. More specifically, the second rank calculating unit 704 assigns a lower rank to any specific set when the energy Energy₁ thereof is higher than that of the other sets.

More specifically, for any specific set, the second ranking calculating unit 704 assigns a higher rank to the specific set than other sets when the energy Energy₁ thereof is the same as that of the other sets while the energy Energyₓ thereof for an excited state that is closer to a specified state, is lower than that of the other sets. More specifically, for any specific set, the second rank calculating unit 704 assigns a lower rank to the specific set than the other sets when the energy Energy₁ thereof is the same as that of the other sets while the energy Energyₓ of an excited state that is closer to the specified state, is higher than that of the other sets. This allows the second rank calculating unit 704 to obtain criteria for determining which candidate is preferable.

The second ranking calculating unit 704 updates, for any one of the candidates, each state based on the set rankings. For example, the second ranking calculating unit 704 updates each state for the candidate with the highest set ranking. This allows the second ranking calculating unit 704 to update each state to a more preferred state, enabling accurate calculation of each state.

The second determining unit 705 determines whether the number of iterations of the energy calculation has exceeded the upper limit corresponding to the excited state Stateₓ that is to be calculated. When the number of energy calculation iterations has not exceeded the upper limit corresponding to the excited state Stateₓ that is to be calculated, the second determining unit 705 performs control such that the functional units such that the second parameter calculating unit 701 to the second rank calculating unit 704 execute the energy calculation again. When the number of iterations of the energy calculation exceeds the upper limit corresponding to the excited state Stateₓ that is to be calculated, the second determining unit 705 terminates the iteration of the energy calculation.

Here, the specific processing performed by the second optimization calculating unit 503 to calculate the energy Energy₂ of the first excited state State₂, which has the next lowest energy after the ground state State₁, is described. The second optimization calculating unit 503 repeats the first energy calculation. This calculation uses quantum chemistry to compute the first excited state State₂ based on the value of the first parameter P₂, while varying the value of the first parameter P₂ representing the first excited state State₂.

The second parameter calculating unit 701 sets an initial value for the value of the first parameter P₂. The second state calculating unit 702 calculates the first excited state State₂ based on the value of the first parameter P₂. The second state calculating unit 702 generates candidates representing sets of mutually orthogonal states based on the ground state State₁ and the calculated first excited state State₂.

The second state calculating unit 702, fixes the ground state State₁ and generates, for example, a candidate 1 representing the set of the first excited state State₂' and the ground state State₁, the first excited state State₂' being obtained by subtracting from the first excited state State₂, a projection component obtained by projecting the first excited state State₂ onto the ground state State₁. The second state calculating unit 702, for example, fixes the first excited state State₂ and generates candidate 2 representing the set of the ground state State₁' and the first excited state State₂, the ground state State₁' being obtained by subtracting from the ground state State₁, a projection component obtained by projecting the ground state State₁ onto the first excited state State₂.

The second energy calculating unit 703 calculates the sets of energies Energy₁ and Energy₂ for each state State₁ and State₂ in each generated candidate. The second energy calculating unit 703 calculates, for example, the set of energy Energy₁ for the ground state State₁ and energy Energy₂' for the first excited state State₂' in candidate 1. The second energy calculating unit 703 calculates, for example, the set of energy Energy₁' for the ground state State₁' and energy Energy₂ for the first excited state State₂ in candidate 2.

The second rank calculating unit 704 sets the rank for the sets of energies Energy₁ and Energy₂ for each candidate such that a point corresponding to any of the sets of energies Energy₁ and Energy₂ relatively closer to the origin in the first coordinate system has a relatively higher rank. The second rank calculating unit 704 updates each state State₁ and State₂ with one of the candidates, based on the set rankings.

The second determining unit 705 determines whether the number of iterations of the first energy calculation has exceeded an upper limit 1 corresponding to the first excited state State₂. The second determining unit 705 controls each functional unit so that, for example, when the number of iterations of the first energy calculation has not exceeded the upper limit 1, the second parameter calculating unit 701 to the second rank calculating unit 704 execute the first energy calculation again. The second determining unit 705 terminates the iteration of the first energy calculation when the number of iterations of the first energy calculation has exceeded the upper limit 1.

The second parameter calculating unit 701 varies the values of the first parameter P₂ in the first energy calculation performed for the second and subsequent executions. The second parameter calculating unit 701 varies the values of the first parameter P₂ so that the ranking of the set of energies Energy₁, Energy₂ of the updated states State₁, State₂ increases in the first coordinate system. This enables the second optimization calculating unit 503 to accurately calculate the first excitation state State₂ such that the constraint that the states State₁ and State₂ are orthogonal with each other I satisfied. The second optimization calculating unit 503 can improve the accuracy of the ground state State₁ when calculating the first excited state State₂.

Here, the specific processing performed by the second optimization calculating unit 503 to calculate the energy Energyₓ of the next lowest energy excited state Stateₓ following the previously calculated excited state State₍ₓ₋₁₎ is described. The second optimization calculating unit 503 repeats a second energy calculation. This calculation uses quantum chemistry to compute the excited state Stateₓ that is to be calculated, based on the value of the second parameter Pₓ. The calculation is performed while varying the values of the second parameter Pₓ representing the excited state Stateₓ that is to be calculated.

The second parameter calculating unit 701 sets an initial value for the second parameter Pₓ. The second state calculating unit 702 calculates the excited state Stateₓ that is to be calculated, based on the values of the second parameter Pₓ. The second state calculating unit 702 generates candidates representing sets of mutually orthogonal states State₁ to Stateₓ based on each state State₁ to Stateₓ including from the ground state State₁ to an excited state State₍ₓ₋₁₎ and the calculated excited state Stateₓ. The second state calculating unit 702 generates one or more candidates, for example, for each state Stateₖ, by fixing Stateₖ and subtracting the projection component of Stateₖ from at least one of the other states Stateⱼ (j≠k) such that the states become orthogonal with each other. The projection component of Stateₖ is obtained by projecting Stateₖ onto the other states Stateⱼ.

The second energy calculating unit 703 calculates the sets of energies Energy₁ to Energyₓ for each state State₁ to Stateₓ in each generated candidate. The second ranking calculating unit 704 sets the ranks for the sets of energies Energy₁ to Energyₓ for each candidate such that a point corresponding to any of the sets of energies Energy₁ to Energyₓ relatively closer to the origin in the second coordinate system has a relatively higher rank. The second rank calculating unit 704 updates each state State₁ to Stateₓ to one of the candidates based on the set priorities.

The second determining unit 705 determines whether the number of iterations of the second energy calculation has exceeded an upper limit x corresponding to the target excited state Stateₓ. The second determining unit 705 performs control for each functional unit so that, for example, when the number of iterations of the second energy calculation has not exceeded the upper limit x, the second parameter calculating unit 701 to the second rank calculating unit 704 execute the second energy calculation again. When the number of iterations of the second energy calculation exceeds the upper limit x, the second determining unit 705 terminates the iteration of the second energy calculation.

The second parameter calculating unit 701 varies the values of the second parameter Pₓ in the second and subsequent second energy calculations. The second parameter calculating unit 701 changes the values of the second parameter Pₓ such that the ranking of the updated set of each state State₁ to Stateₓ and the corresponding energy Energy₁ to Energyₓ thereof in the second coordinate system becomes higher. The second parameter calculating unit 701, for example, searches within a preset range for values of parameter Pₓ that will result in a higher ranking for the sets of energies Energy₁ to Energyₓ in the next second energy calculation. The range is, for example, specified by the parameter range 402 included in the energy calculation setting information 400.

Specifically, during the search, the second parameter calculating unit 701 may reference each candidate generated by the second state calculating unit 702 and each set of energies Energy₁ to Energyₓ calculated by the second energy calculating unit 703. This enables the second optimization calculating unit 503 to accurately calculate the excited state Stateₓ that is to be calculated, while satisfying the constraint that the state State₁ to Stateₓ are orthogonal with each other. The second optimization calculating unit 503 can improve the accuracy from the ground state State₁ to the excited state Stateₓ₋₁ when calculating the target excited state Stateₓ.

Here, the description of Fig. 5 is continued. The output unit 504 outputs the processing results of at least one of the functional units. The output format includes, for example, display on a monitor, printing output to a printer, transmission to an external device via network I/F 303, or storage in a memory area such as memory 302 or recording medium 305. This enables output unit 504 to notify the user of the processing results from at least one functional unit, thereby improving the convenience of excitation energy calculating device 201.

The output unit 504 outputs in a manner enabling user reference, for example, the energies Energy₁ to Energyₓ of each state State₁ to Stateₓ last updated by the second optimization calculating unit 503, after specific energy calculations are repeatedly performed by the second optimization calculating unit 503. The output unit 504 may transmit the energies Energy₁ to Energyₓ for each state State₁ to Stateₓ last updated by the second optimization calculating unit 503 to another computer, for example, after the second optimization calculating unit 503 repeats specific energy calculations. This allows the output unit 504 to make the energy values Energy₁ to Energyₓ for each state State₁ to Stateₓ available externally.

The output unit 504 may, for example, output the sets of each state State₁ to Stateₓ and each the energies Energy₁ to Energyₓ corresponding thereto in a manner enabling user reference. The output unit 504 may, for example, output each state State₁ to Stateₓ and each the energy Energy₁ to Energyₓ thereof to other computers. This allows the output unit 504 to make each energy Energy₁ to Energyₓ of each state available externally.

Furthermore, when outputting each state State₁ to Stateₓ and the size of any state State₁ to Stateₓ is too large, the output unit 504 may output only the non-zero portions by outputting the row number, column number, and state data thereof.

Furthermore, the functional components of the excitation energy calculating device 201 may be implemented, for example, by multiple computers in the information processing system 200. The multiple computers may be, for example, the excitation energy calculating device 201 and the client device 202. In this case, communication between functional components on different computers is performed, for example, by transmission and reception between functional components via the network 210.

### (First Operation Example of Excitation Energy Calculating Device 201)

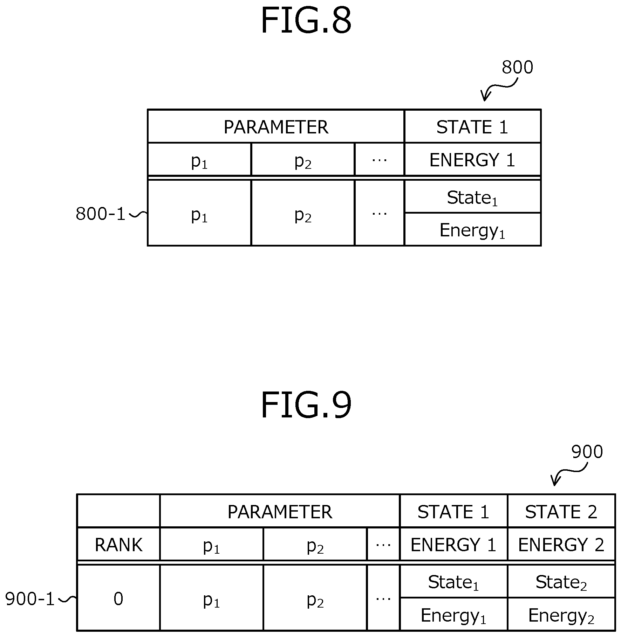

Next, with reference to Figs. 8 to 13, a first operation example of the excitation energy calculating device 201 is described. In the first operation example, Ns=2 is assumed. First, with reference to Fig. 8, stored contents of a first calculation result table 800 are described. Specifically, the first calculation result table 800 stores the calculation results of specific energy calculations that the excitation energy calculating device 201 repeatedly performs when calculating the ground state.

Fig. 8 is an explanatory diagram depicting an example of the stored contents of the first calculation result table 800. In Fig. 8, the first calculation result table 800 has fields for a parameter, state 1, and energy 1. Information is stored in each field, whereby the calculation result is stored as a record 800-a. Here, a is an arbitrary integer.

The parameter field stores the values p₁, p₂, ..., p_{Np} of parameter P₁ representing the ground state. Here, Np is the number of values forming parameter P₁. The state 1 field stores the ground state State₁ of the substance. In the field for energy 1, the energy Energy₁ of the ground state State₁ of the substance is set.

The excitation energy calculating device 201 calculates the ground state State₁ and the energy Energy₁ of the ground state State₁ by repeatedly performing specific energy calculations while using the first calculation result table 800. First, the first parameter calculating unit 601, specifically in the first execution of the specific energy calculations, sets initial values for the parameter values p₁, p₂, ..., p_{Np} representing the ground state and stores the initial values to the first calculation result table 800. The initial values may, for example, be randomly selected.

The first state calculating unit 602 specifically calculates the ground state State₁ based on the values of parameter P₁ using quantum chemistry calculations in the first execution of the specific energy calculations and stores the results to the first calculation result table 800. The first energy calculating unit 603 specifically calculates the energy Energy₁ of the calculated ground state State₁ in the first execution of the specific energy calculations and stores the result to the first calculation result table 800.

The first determining unit 604 specifically determines whether the number of iterations of the specific energy calculations has exceeded the upper limit 1 for the ground state State₁. Specifically, when the number of iterations of the specific energy calculations has not exceeded the upper limit 1, the first determining unit 604 performs control so that the functional units (the first parameter calculating unit 601 to the first energy calculating unit 603) execute the specific energy calculations again. Specifically, when the number of iterations of the specific energy calculations has exceeded the upper limit 1, the first determining unit 604 terminates the iteration of the specific energy calculation.

The first parameter calculating unit 601, specifically, in the second and subsequent energy calculations, changes the values of parameter P₁ so that the energy Energy₁ of the ground state State₁ becomes lower, and stores the results to the first calculation result table 800. The first state calculating unit 602 specifically calculates the ground state State₁ based on the values of parameter P₁ using quantum chemistry calculations in the second and subsequent execution of the specific energy calculation, and stores the results to the first calculation result table 800. The first energy calculating unit 603 specifically calculates the energy Energy₁ of the calculated ground state State₁ in the second and subsequent executions of the specific energy calculations, and stores the results to the first calculation result table 800.

Here, the excitation energy calculating device 201 is assumed to repeatedly performed specific energy calculations, calculated the ground state State₁, and calculated the energy Energy₁ of the ground state State₁.

Next, with reference to Fig. 9, contents stored in a second calculation result table 900 are described. Specifically, the second calculation result table 900 stores the calculation results of the specific energy calculations that the excitation energy calculating device 201 repeatedly performs when calculating the ground state.

Fig. 9 is an explanatory diagram depicting an example of the stored contents of the second calculation result table 900. In Fig. 9, the second calculation result table 900 has fields for parameters, state 1, energy 1, state 2, energy 2, and rank. Information is set in each field, whereby the second calculation result table 900 stores calculation results as a record 900-b. Here, b is an arbitrary integer.

The parameter field stores the values p₁, p₂, ..., p_{Np} of parameter P₂ representing the first excited state. Np is the number of values forming parameter P₂. The state 1 field stores the ground state State₁ of the substance. The field for energy 1 is set to the energy Energy₁ of the ground state of the substance State₁.

In the field for State 2 , the first excited state State₂ is set. In the field for Energy 2, the energy Energy₂ of the first excited state State₂ is set. In the ranking field, a ranking is set that indicates the order of magnitude of the evaluation for the set including the energy Energy₁ of the ground state State₁ and the energy Energy₂ of the first excited state State₂. The ranking indicates, for example, that a smaller value represents a better evaluation.

The excitation energy calculating device 201 calculates the first excited state State₂ and the energy Energy₂ of the first excited state State₂ by repeatedly performing specific energy calculations using the second calculation result table 900. Specifically, in the first execution of the specific energy calculations, the second parameter calculating unit 701 sets initial values for the parameter values p₁, p₂, ..., p_{Np} representing the first excited state State₂ and stores the values to the second calculation result table 900. The initial values may, for example, be randomly selected.

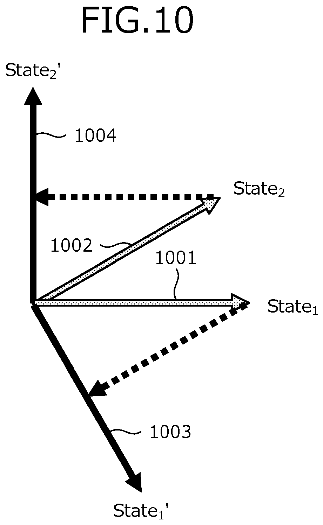

The second state calculating unit 702 specifically calculates the first excited state State₂ based on the values of parameter P_{2,} using quantum chemistry calculations, in the first execution of the specific energy calculations. Specifically, the second state calculating unit 702, in the first execution of the specific energy calculations, obtains the ground state State₁ by referring to the first calculation result table 800. Specifically, the second state calculating unit 702, based on the ground state State₁ and the calculated first excited state State₂, generates candidates representing sets of mutually orthogonal states State₁ and State₂. Here, the description shifts to Fig. 10, which depicts an example of the excitation energy calculating device 201 generating such candidates.

Fig. 10 is an explanatory diagram depicting an example of generating candidates. In Fig. 10, the ground state State₁ corresponds to vector 801. The first excited state State₂ corresponds to vector 802. As depicted in Fig. 10, the second state calculating unit 702 specifically fixes the ground state State₁ and generates the first excited state State₂' represented by vector 804 and obtained by subtracting from the first excited state State₂, a projection component of the first excited state₂ obtained by projecting the first excited state₂ onto the ground state State₁. Specifically, the second state calculating unit 702 generates candidate 1 representing the set of the first excited state State₂' and the ground state State₁.

Furthermore, the second state calculating unit 702, for example, fixes the first excited state State₂ and generates the ground state State₁' represented by a vector 803 and obtained by subtracting from the ground state State₁, a projection component obtained by projecting the ground state State₁ onto the first excited state State₂. The second state calculating unit 702 generates, for example, candidate 2 representing the set of the ground state State₁' and the first excited state State₂.

The second energy calculating unit 703 calculates the sets of energies Energy₁ and Energy₂ for each state State₁ and State₂ in each generated candidate. The second energy calculating unit 703, for candidate 1, calculates, for example, a set 1 including the energy Energy₁ of the ground state State₁ and the energy Energy₂' of the first excited state State₂'. The second energy calculating unit 703, for candidate 2, calculates, for example, a set 2 including the energy Energy₁' of the ground state State₁' and the energy Energy₂ of the first excited state State₂.

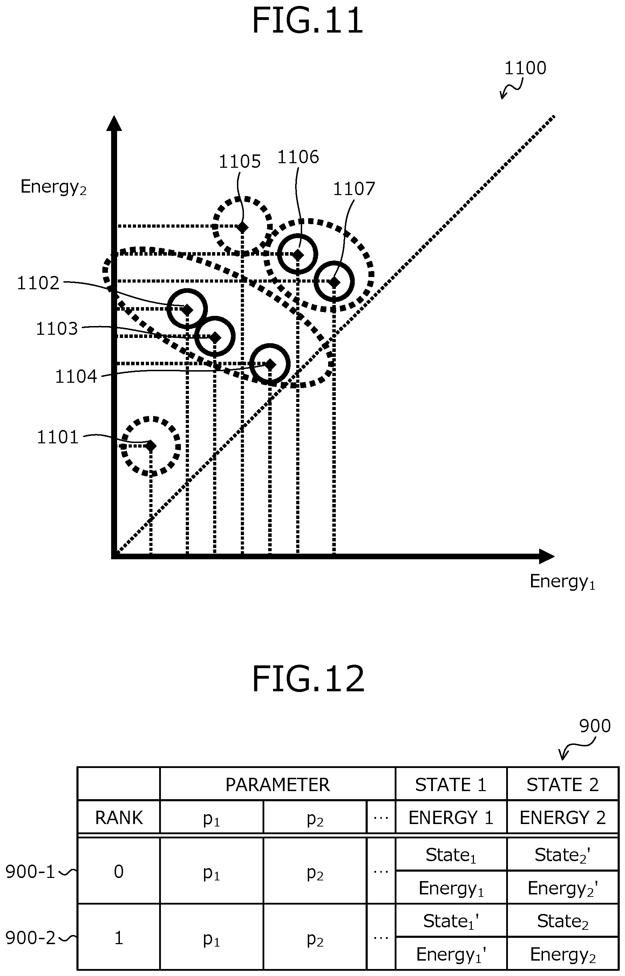

The second rank calculating unit 704 sets a first coordinate system having axes representing the energies Energy₁ and Energy₂ of each of states State₁ and State₂. The second rank calculating unit 704 sets the rank for the sets of energies Energy₁ and Energy₂ for each candidate such that a point corresponding to any of the sets of energies Energy₁ and Energy₂ relatively closer to the origin in the first coordinate system has a relatively higher rank. Here, the second ranking calculating unit 704 sets a rank of 1 for candidate 1 and a rank of 2 for candidate 2 and stores the rankings to the second calculation result table 900. Now, moving to the description of Fig. 11, an example of how the excitation energy calculating device 201 sets the ranks is described.

Fig. 11 is an explanatory diagram depicting an example of setting rankings. The example in Fig. 11 describes a case where seven candidates exist, each representing a set of states State₁ to Stateₓ. Here, 2≤x≤Ns is assumed. The second ranking calculating unit 704 sets a coordinate system 1100 including axes representing the energies Energy₁ to Energyₓ of each of the states State₁ to Stateₓ in a coordinate system 1100. In the example depicted in Fig. 11, for convenience, the coordinate system 1100 is depicted as a two-dimensional space containing axes representing the energies Energy₁ to Energyₓ. The set of Energy₁ to Energyₓ of each candidate corresponds to any one of points 1101 to 1107 in the coordinate system 1100.

The second rank calculating unit 704 sets the rank for the set of energies Energy₁ and Energy₂ in each candidate such that a point corresponding to a set of energies Energy₁ to Energyₓ relatively closer to the origin in the coordinate system 1100 has a relatively higher rank. The second rank calculating unit 704 calculates, for example, a distance between one point corresponding to a set of energies Energy₁ to Energyₓ for each candidate and the origin in the coordinate system 1100.

The distance is defined, for example, by the number of other points where all energies Energy₁ to Energyₓ are lower than those of the one point. The distance may, for example, be the Euclidean distance. The second calculation result table 900 may, for example, include a distance field. The distance field stores the distance between the point corresponding to the set of energies Energy₁ to Energyₓ of each candidate and the origin. The second ranking calculating unit 704 may store the calculated distances to the second calculation result table 900.

In the example depicted in Fig. 11, specifically, in the coordinate system 1100, the number of other points where all energies Energy₁ to Energyₓ are lower than those of point 1101 is zero. Therefore, the second ranking calculating unit 704 sets the distance between point 1101 and the origin as "0". Also, specifically, in the coordinate system 1100, the number of other points where all Energy₁ to Energyₓ are lower than points 1102 to 1104 is 1. Therefore, the second ranking calculating unit 704 sets the distances between points 1102 to 1104 and the origin to "1".

Specifically, in the coordinate system 1100, the number of other points lower than point 1105 in all Energy₁ to Energyₓ is 2. Therefore, the second rank calculating unit 704 sets the distance between point 1105 and the origin to "2". Specifically, in the coordinate system 1100, the number of other points lower than points 1106 and 1107 in all energies Energy₁ to Energyₓ is 3. Therefore, the second rank calculating unit 704 sets the distance between each of points 1106 and 1107 and the origin to "3". The second rank calculating unit 704 sets the rank for each set of Energy₁ to Energyₓ among the candidates such that a candidate having a relatively smaller set distance has a relatively higher rank.

Here, for example, it is possible that multiple points having the same distance from the origin are present. In this case, the second ranking calculating unit 704 assigns different rankings to each set of Energy₁ to Energyₓ that correspond to the multiple points with the same distance from the origin. Specifically, the second rank calculating unit 704 selects the energy Energy₁ to Energyₓ from the lowest value in ascending order until distinct priorities have been assigned to each set including Energy₁ to Energyₓ corresponding to the multiple points. Specifically, the second rank calculating unit 704 assigns different priorities to each set of energies Energy₁ to Energyₓ corresponding to multiple points having the same distance from the origin, such that a relatively lower selected energy Energyᵢ has a relatively higher rank. The second ranking calculating unit 704 may also assign the same rank to each set of energies Energy₁ to Energyₓ corresponding to multiple points having the same distance from the origin.

In the example depicted in Fig. 11, the second rank calculating unit 704 sets rank "1" for the set of energies Energy₁ to Energyₓ corresponding to point 1101. The second rank calculating unit 704 sets rank "2" for the set of energies Energy₁ to Energyₓ corresponding to point 1102. The second rank calculating unit 704 sets rank "3" for the set of energies Energy₁ to Energyₓ corresponding to point 1103.

The second rank calculating unit 704 sets rank "4" for the set of energies Energy₁ to Energyₓ corresponding to point 1104. The second rank calculating unit 704 sets rank "5" for the set of energies Energy₁ to Energyₓ corresponding to point 1105. The second rank calculating unit 704 sets rank "6" for the set of energies Energy₁ to Energyₓ corresponding to point 1106. The second rank calculating unit 704 sets the rank "7" for the set of energies Energy₁ to Energyₓ corresponding to point 1107. Here, with reference to Fig. 12, the contents stored in the second calculation result table 900 after the excitation energy calculating device 201 saves various information will be described.

Fig. 12 is an explanatory diagram depicting an example of the stored contents of the second calculation result table 900 after various information is stored. As depicted in Fig. 12, the second calculation result table 900 stores a set including the ground state State₁ and the first excited state State₂', as candidate 1. The second calculation result table 900 stores a set 1 including the energy Energy₁ of the ground state State₁ and the energy Energy₂' of the first excited state State₂', for candidate 1. The second calculation result table 900 stores rank 1 for set 1.

The second calculation result table 900 stores a set including the ground state State₁' and the first excited state State₂ as candidate 2. The second calculation result table 900 stores a set 2 including the Energy₁' of the ground state State₁' and the Energy₂ of the first excited state State₂ as candidate 2. The second calculation result table 900 stores rank 2 for set 2.

In Fig. 12, the second ranking calculating unit 704 refers to the second calculation result table 900 and in the second calculation result table 900, updates and saves each state State₁ and State₂ with the higher-ranked candidate 1 based on the set ranking. The second determining unit 705 determines whether the number of iterations of a specific energy calculation has exceeded the upper limit 2 corresponding to the first excited state State₂.

When the number of iterations of the specific energy calculations has not exceeded the upper limit 2, the second determining unit 705 controls the functional units so that the second parameter calculating unit 701 to the second rank calculating unit 704 execute the specific energy calculations again. When the number of iterations of the specific energy calculations exceeds the upper limit 2, the second determining unit 705 terminates the iteration of the specific energy calculation.

The second parameter calculating unit 701 changes the values of parameter P₂ in the second and subsequent executions of the specific energy calculations. The second parameter calculating unit 701 changes the values of parameter P₂ so that the rank of the updated sets of states State₁ and State₂ and the energies Energy₁ and Energy₂ thereof in the first coordinate system increases.

The second parameter calculating unit 701 searches for values of parameter P₂ within a preset range such that the ranking for the set of energies Energy₁ and Energy₂ of states State₁ and State₂, calculated in the next specific energy calculation, becomes higher. The range is determined from the parameter range 402 included in the energy calculation setting information 400. Specifically, during the search, the second parameter calculating unit 701 may refer to the set 1 and rank 1 for the set 1, and the set 2 and rank 2 for the set 2.

The search is implemented using an optimization algorithm. Any existing optimization technique may be used. Examples of optimization algorithms include the Nelder-Mead method, Powell conjugate direction method, or the Broyden-Fletcher-Goldfarb-Shanno algorithm (BFGSA) method. Furthermore, optimization algorithms include, for example, the Sequential Least Squares Programming (SLSQP) method, simulated annealing, genetic algorithms, differential evolution algorithms, and particle swarm optimization.

The excitation energy calculating device 201 repeatedly performs specific energy calculations, calculates the first excited state State₂, and calculates the energy Energy₂ of the first excited state State₂. At this time, the excitation energy calculating device 201 updates the ground state State₁ and updates the energy Energy₁ of the ground state State₁.

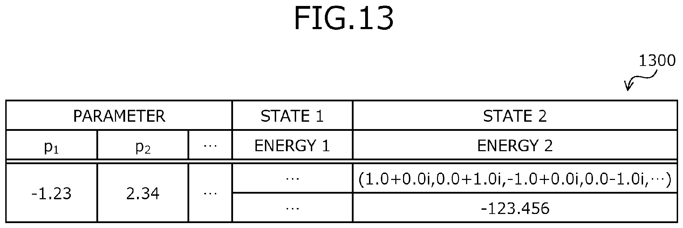

Thus, the second optimization calculating unit 503 can accurately calculate the first excited state State₂ such that the constraint that states State₁ and State₂ are orthogonal to each other is satisfied. The second optimization calculating unit 503 can improve the accuracy of the ground state State₁ when calculating the first excited state State₂. Next, the description moves to Fig. 13, which depicts an example of the excitation energy calculating device 201 outputting calculation results.

Fig. 13 is an explanatory diagram depicting an example of outputting calculation results. In Fig. 13, the output unit 504 generates an output result table 1300 which corresponds to the latest updated states State₁ and State₂ by the second optimization calculating unit 503 with the energies Energy₁ and Energy₂ of the states State₁ and State₂, respectively. The output unit 504 outputs the generated output result table 1300 for the user to reference.

Output result table 1300 has the values p₁, p₂, ..., p_{Np} of parameter P₂, states State₁ and State₂, and the energies Energy₁, Energy₂ of states State₁ and State₂, respectively. "State 1" in Fig. 13 corresponds to state State₁. "State 2" in Fig. 13 corresponds to state State₂. "Energy 1" in Fig. 13 corresponds to energy Energy₁. "Energy 2" in Fig. 13 corresponds to energy Energy₂.

In the example depicted in Fig. 13, only some values of the output result table 1300 are depicted specifically. For example, the value p₁ of parameter P₂ is "-1.23". The value p₂ of parameter P₂ is "2.34". Furthermore, state 2 (State₂) is "(1.0+0.0i, 0.0+1.0i, -1.0+0.0i, 0.0-1.0i, ...)". Also, Energy 2 (Energy₂) is "-123.456".

According to output result table 1300, for example, the analyst can determine not only the energy of the ground state (State 1) for the substance-under-analysis, but also the energy of the first excited state (State 2). This enables the analyst to analyze properties such as whether the substance will react or how readily the reaction will proceed.

The first operation example described the case where the excitation energy calculating device 201 calculates the ground state State₁ and the energy Energy₁, but configuration is not limited to this example. For example, in the first operation example, the excitation energy calculating device 201 may accept setting of initial values for the ground state State₁ and the energy Energy₁. In this case, the excitation energy calculating device 201 needs not calculate the ground state State₁ and the energy Energy₁.

### (Second Operation Example of Excitation Energy Calculating Device 201)

Next, another example, a second operation example, of the excitation energy calculating device 201 is described. In the second operation example, Ns=3 is assumed. In the second operation example, as in the first operation example, the excitation energy calculating device 201 is assumed to have calculated the energy Energy₂ of the first excited state State₂. Furthermore, the excitation energy calculating device 201 updates the ground state State₁ and updates the energy Energy₁ of the ground state State₁. The excitation energy calculating device 201 stores a third calculation result table similar to the second calculation result table.

The excitation energy calculating device 201 calculates the second excited state State₃ and the energy Energy₃ of the second excited state State₃ by repeatedly performing specific energy calculations while utilizing the third calculation result table, as in the first operation example. The second parameter calculating unit 701 performs processing similar to that in the first operation example. The second state calculating unit 702 performs processing similar to that in the first operation example. The second state calculating unit 702 generates candidates representing sets of mutually orthogonal states State₁ to State₃, based on each state State₁ to State₃, for example.

The second state calculating unit 702 specifically fixes the ground state State₁ and generates the first excited state State₂(1), which is the first excited state State₂ minus the projection component obtained by projecting the first excited state State₂ onto the ground state State₁. The second state calculating unit 702, specifically, fixes the ground state State_{1,} and generates the second excited state State₃(1) by subtracting from the second excited state State₃, projection components obtained by projecting the second excited state State₃ onto the ground state State₁ and the first excited state State₂(1). Specifically, the second state calculating unit 702 generates candidate 1 representing the set including the ground state State₁, the first excited state State₂(1), and the second excited state State₃(1).

The second state calculation unit 702, specifically, fixes the ground state State₁ and generates the second excited state State₃(2) obtained by subtracting from the second excited state State₃, projection components obtained by projecting the second excited state State₃ onto the ground state State₁. Specifically, the second state calculating unit 702 fixes the ground state State₁ and generates the first excited state State₂(2) by subtracting from the first excited state State₂, projection components obtained by projecting the first excited state State₂ onto the ground state State₁ and the second excited state State₃(2). Specifically, the second state calculating unit 702 generates candidate 2 representing the set including the ground state State₁, the first excited state State₂(2), and the second excited state State₃(2).

The second state calculating unit 702 specifically fixes the first excited state State₂ and generates the ground state State₁(3) by subtracting from the ground state State₁, a projection component obtained by projecting the ground state State₁ onto the first excited state State₂. The second state calculating unit 702, specifically, fixes the first excited state State₂ and generates a second excited state State₃(3) by subtracting from the second excited state State₃, projection components obtained by projecting the second excited state State₃ onto the ground state State₁(3) and the first excited state State₂. Specifically, the second state calculating unit 702 generates candidate 3 representing the set including the ground state State₁(3), the first excited state State₂, and the second excited state State₃(3).

The second state calculating unit 702 specifically fixes the first excited state State₂ and generates the second excited state State₃(4) by subtracting from the second excited state State₃, a projection component obtained by projecting the second excited state State₃ onto the first excited state State₂. Specifically, the second state calculating unit 702, fixes the first excited state State₂ and generates the ground state State₁(4) by subtracting from the ground state State₁, projection components obtained by projecting the ground state State₁ onto the second excited state State₃(4) and the first excited state State₂. Specifically, the second state calculating unit 702 generates candidate 4 representing the set including the ground state State₁(4), the first excited state State₂, and the second excited state State₃(4).

The second state calculating unit 702 specifically fixes the second excited state State₃ and generates the ground state State₁(5) by subtracting from the ground state State₁, a projection component obtained by projecting the ground state State₁ onto the second excited state State₃. Specifically, the second state calculating unit 702 fixes the second excited state State₃ and generates the first excited state State₂(5) by subtracting from the first excited state State₂, projection components obtained by projecting the first excited state State₂ onto the ground state State₁(5) and the second excited state State₃. Specifically, the second state calculating unit 702 generates candidate 5 representing the set including the ground state State₁(5), the first excited state State₂(5), and the second excited state State₃.

The second state calculating unit 702 specifically fixes the second excited state State₃ and generates the first excited state State₂(6) by subtracting from the first excited state State₂, a projection component obtained by projecting the first excited state State₂ onto the second excited state State₃. The second state calculating unit 702 specifically fixes the second excited state State₃ and generates the ground state State₁(6) by subtracting from the ground state State₁, projection components obtained by projecting the ground state State₁ onto the first excited state State₂(6) and the second excited state State₃. Specifically, the second state calculating unit 702 generates candidate 4 representing the set including the ground state State₁(6), the first excited state State₂(6), and the second excited state State₃.

The second energy calculating unit 703 performs processing similar to that in the first operation example. The second rank calculating unit 704 performs processing similar to that in the first operation example. The second determining unit 705 performs processing similar to that in the first operation example. This enables the second optimization calculating unit 503 to accurately calculate the second excited state State₃ under the constraint that the ground excited state State₁ to the second excited state State₃ are orthogonal to each other. The second optimization calculating unit 503 can improve the accuracy of the ground state State₁ and the first excited state State₂ when calculating the second excited state State₃.

The excitation energy calculating device 201 can calculate the energy Energy_{Ns} of the Ns-th excited state State_{Ns} even when Ns≥4, in the same manner as in the second operation example.

The second operation example described the case where the excitation energy calculating device 201 references the ground state State₁ and energy Energy₁, the first excited state State₂, and energy Energy₂ calculated or updated as in the first operation example, but configuration is not limited to this case.

For example, in the second operation example, the excitation energy calculating device 201 may accept setting of initial values for the ground state State₁, the energy Energy₁, the first excited state State₂, and the energy Energy₂. In this case, the excitation energy calculating device 201 needs not calculate or update the ground state State₁, the energy Energy₁, the first excited state State₂, and the energy Energy₂, as in the first operation example.

Similarly, when Ns≥4, the excitation energy calculating device 201 may accept setting initial values from the ground state State₁ to the (Ns-1)-th excited state State_{Ns-1} and energies Energy₁ to Energy_{Ns-1}.

As depicted in the first operation example and the second operation example, the excitation energy calculating device 201 can accurately calculate the Ns-th excited state State_{Ns} such that the constraint that states State₁ to State_{Ns} are orthogonal to each other is satisfied. In doing so, the excitation energy calculating device 201 can improve the accuracy of each state State₁ to State_{Ns-1} when calculating the Ns-th excitation state State_{Ns}.

For example, conventional methods have a problem in that the Ns-th excited state State_{Ns} cannot be accurately calculated unless the parameters related to the degree of overlap between states included in the objective function are appropriately selected. The conventional method is, for example, Variational Quantum Computation of Excited States. In contrast, the excitation energy calculating device 201 does not utilize parameters related to the degree of overlap between states included in the objective function. This enables precise calculation of the Ns-th excited state State_{Ns} while reducing the workload on the analyst.

Furthermore, for example, the conventional methods have a problem in that it may not be possible to calculate each state State₁ to State_{Ns} such that the constraint of states being mutually orthogonal is satisfied. In contrast, the excitation energy calculating device 201 can satisfy the constraint that states State₁ to State_{Ns} are mutually orthogonal.

Furthermore, for example, in the conventional methods, when multiple excited states are analyzed sequentially from the lowest energy and the preceding excited states cannot be calculated accurately, it becomes impossible to accurately calculate the subsequent excited states. In contrast, the excitation energy calculating device 201 can improve the accuracy of the preceding states State₁ to State_{(Nx-1)} when calculating a subsequent x-th excited state State_{Nx}. Consequently, the excitation energy calculating device 201 can accurately calculate the entire set of states State₁ to State_{Ns}.

### (Overall Processing Procedure)

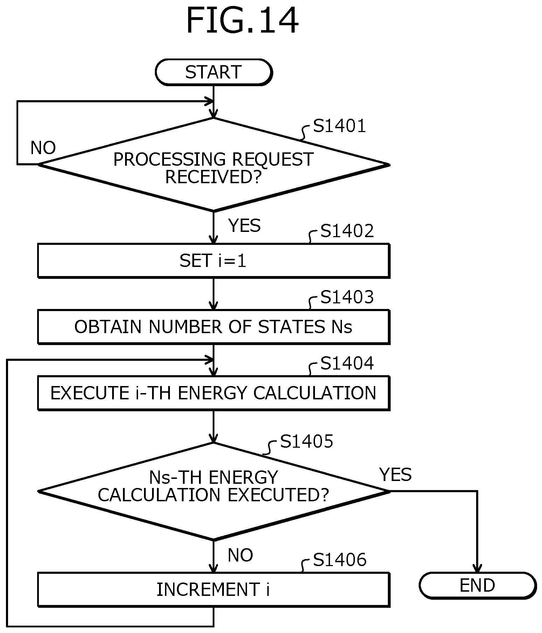

Next, with reference to Fig. 14, an example of the overall processing procedure executed by the excitation energy calculating device 201 is described. The overall processing is realized, for example, by the CPU 301 depicted in Fig. 3, storage areas such as the memory 302 and the recording medium 305, and the network I/F 303.

Fig. 14 is a flowchart depicting an example of the overall processing procedure. In Fig. 14, the excitation energy calculating device 201 determines whether a processing request has been received from the client device 202 (step S1401). When a processing request has not been received (step S1401: NO), the excitation energy calculating device 201 returns to the process at step S1401. On the other hand, when a processing request has been received (step S1401: YES), the excitation energy calculating device 201 proceeds to the process at step S1402.

At step S1402, the excitation energy calculating device 201 sets i=1 (step S1402). The excitation energy calculating device 201 obtains the number of states Ns based on the received processing request (step S1403). Next, the excitation energy calculating device 201 proceeds to the process at step S1404.

At step S1404, the excitation energy calculating device 201 executes the i-th energy calculation process, calculates the i-th state Sᵢ, and calculates the energy Eᵢ of the i-th state Sᵢ (step S1404).

The excitation energy calculating device 201 determines whether the excitation energy calculating device 201 has executed up to the Ns-th energy calculation process (step S1405). Here, when excitation energy calculating device 201 has not executed up to the Ns-th energy calculation process (step S1405: NO), the excitation energy calculating device 201 increments i (step S1406) and returns to the process at step S1404. On the other hand, when the Ns-th energy calculation process has been executed (step S1405: YES), the excitation energy calculating device 201 terminates the overall process.

### (First Energy Calculation Processing Procedure)

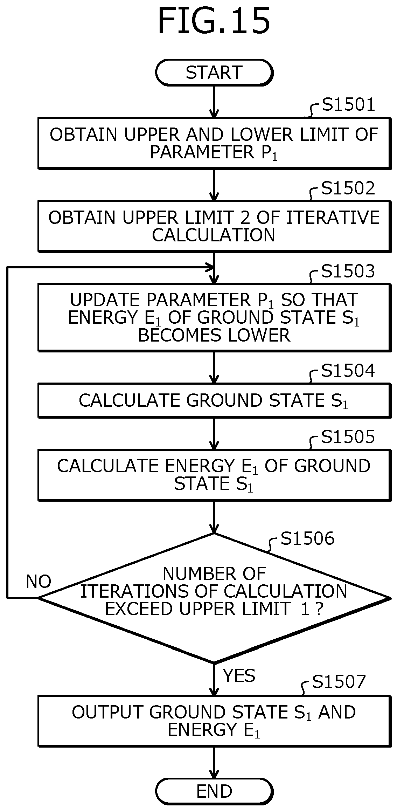

Next, with reference to Fig. 15, an example of a procedure of a first energy calculation process executed by the excitation energy calculating device 201 is described. The first energy calculation processing is realized, for example, by the CPU 301, storage areas such as the memory 302 and the recording medium 305, and the network I/F 303, depicted in Fig. 3.

Fig. 15 is a flowchart depicting an example of a procedure of a first energy calculation process. In Fig. 15, the excitation energy calculating device 201 obtains the upper and lower limits of the parameter P₁ representing the ground state S₁ (step S1501). The excitation energy calculating device 201 obtains the upper limit 1 of the number of iterations (step S1502). The excitation energy calculating device 201 proceeds to the process at step S1503.

At step S1503, the excitation energy calculating device 201 updates the parameter P₁ so that the energy E₁ of the ground state S₁ becomes lower (step S1503). The excitation energy calculating device 201 calculates the ground state S₁ based on the parameter P₁ (step S1504). The excitation energy calculating device 201 calculates the energy E₁ of the ground state S₁, based on the calculated ground state S₁ (step S1505).

The excitation energy calculating device 201 determines whether the number of iterations of calculation has exceeded the upper limit 1 (step S1506). Here, when the number of iterations of calculation has not exceeded the upper limit 1 (step S1506: NO), the excitation energy calculating device 201 returns to the process at step S1503. On the other hand, when the number of iterations of calculation has exceeded the upper limit 1 (step S1506: YES), the excitation energy calculating device 201 proceeds to the process at step S1507.

At step S1507, the excitation energy calculating device 201 outputs the last calculated ground state S₁ and the last calculated energy E₁ of the ground state S₁ (step S1507). The excitation energy calculating device 201 may output the parameter P₁, the last calculated ground state S₁, and the last calculated energy E₁ of the ground state S₁. The excitation energy calculating device 201 ends the first energy calculation process. Thus, the excitation energy calculating device 201 can calculate the ground state S₁ and the energy E₁ of the ground state S₁.

### (Procedure of Second Energy Calculation Process)

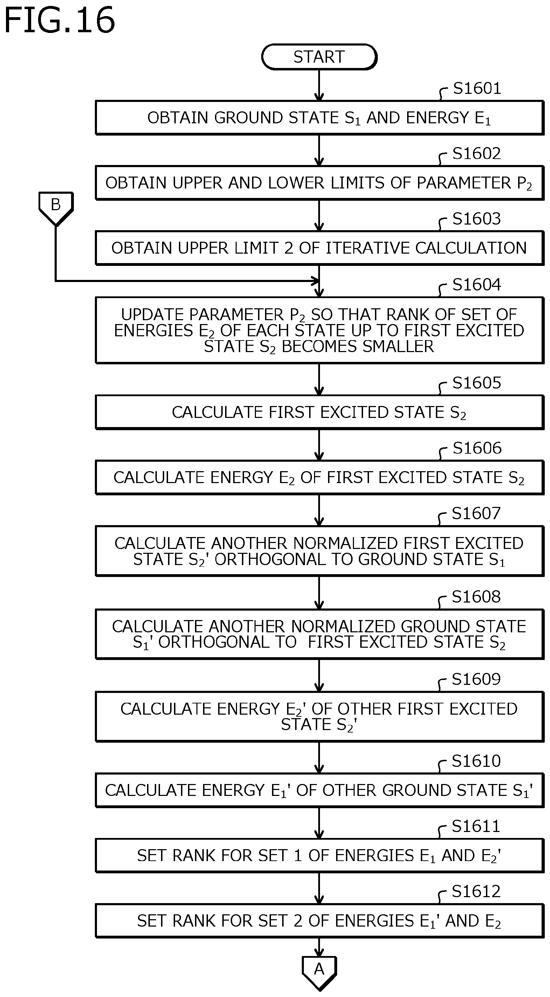

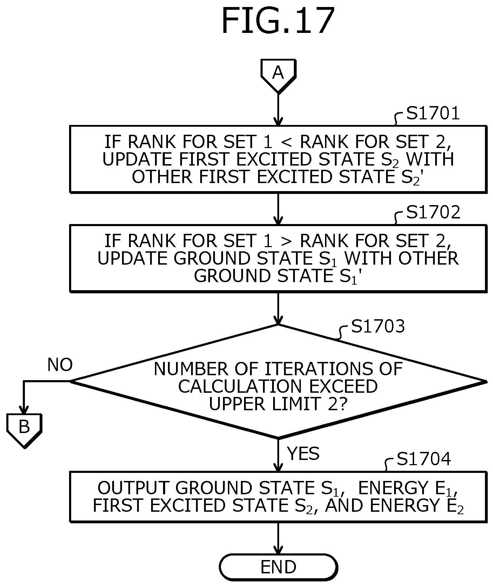

Next, with reference to Figs. 16 and 17, a procedure of a second energy calculation process executed by the excitation energy calculating device 201 is described. The second energy calculation process is realized, for example, by the CPU 301, storage areas such as the memory 302 and the recording medium 305, and the network I/F 303, depicted in Fig. 3.

Figs. 16 and 17 are flowcharts depicting a procedure of the second energy calculation process. In Fig. 16, the excitation energy calculating device 201 obtains the ground state S₁ and the energy E₁ of the ground state S₁ (step S1601).

The excitation energy calculating device 201 obtains the upper and lower limits of the parameter P₂ for representing the first excited state S₂ (step S1602). The excitation energy calculating device 201 obtains the upper limit 2 of the number of iterations of calculation (step S1603). The excitation energy calculating device 201 proceeds to the process at step S1604.

At step S1604, the excitation energy calculating device 201 updates parameter P₂ such that the rank of the set including energies E₂ for each state up to the first excited state S₂ becomes smaller (step S1604). The excitation energy calculating device 201 calculates the first excited state S₂ based on the parameter P₂ (step S1605). The excitation energy calculating device 201 calculates the energy E₂ of the first excited state S₂ based on the calculated first excited state S₂ (step S1606).

The excitation energy calculating device 201 calculates another normalized first excited state S₂' orthogonal to the ground state S₁, based on the first excited state S₂ (step S1607). The excitation energy calculating device 201 calculates another normalized ground state S₁' orthogonal to the first excited state S₂, based on the ground state S₁ (step S1608). The excitation energy calculating device 201 calculates the energy E₂' of the other calculated first excited state S₂' (step S1609). The excitation energy calculating device 201 calculates the energy E₁' of the other calculated ground state S₁' (step S1610).

The excitation energy calculating device 201 sets the rank for the set 1 including the energy E₁ of the ground state S₁ and the energy E₂' of the other normalized first excited state S₂' (step S1611). The excitation energy calculating device 201 sets the rank for the set 2 including the energy E₁' of the other normalized ground state S₁' and the energy E₂ of the first excited state S₂ (step S1612). The excitation energy calculating device 201 proceeds to the process at step S1701 in Fig. 17.

In Fig. 17, when the rank for set 1≤the rank for set 2 is true, the excitation energy calculating device 201 updates the first excited state S₂ with the other normalized first excited state S₂' (step S1701). When the rank for set 1>the rank for set 2 is true, the excitation energy calculating device 201 updates the ground state S₁ with the other normalized ground state S₁' (step S1702).

The excitation energy calculating device 201 determines whether the number of iterations of calculation has exceeded the upper limit 2 (step S1703). Here, when the number of iterations of calculation has not exceeded the upper limit 2 (step S1703: NO), the excitation energy calculating device 201 returns to the process at step S1604 in Fig. 16. On the other hand, when the number of iterations of calculation exceeds the upper limit 2 (step S1703: YES), the excitation energy calculating device 201 proceeds to the process at step S1704.

At step S1704, the excitation energy calculating device 201 outputs the last calculated ground state S₁, the energy E₁ of the ground state S₁, the first excited state S₂, and the energy E₂ of the first excited state S₂ (step S1704). The excitation energy calculating device 201 may also output the parameter P₁, the ground state S₁, the energy E₁ of the ground state S₁, the parameter P₂, the first excited state S₂, and the energy E₂ of the first excited state S₂. The excitation energy calculating device 201 ends the second energy calculation process. Thus, the excitation energy calculating device 201 can calculate the first excited state S₂ and the energy E₂ of the first excited state S₂.

### (i-th Energy Calculation Processing Procedure)

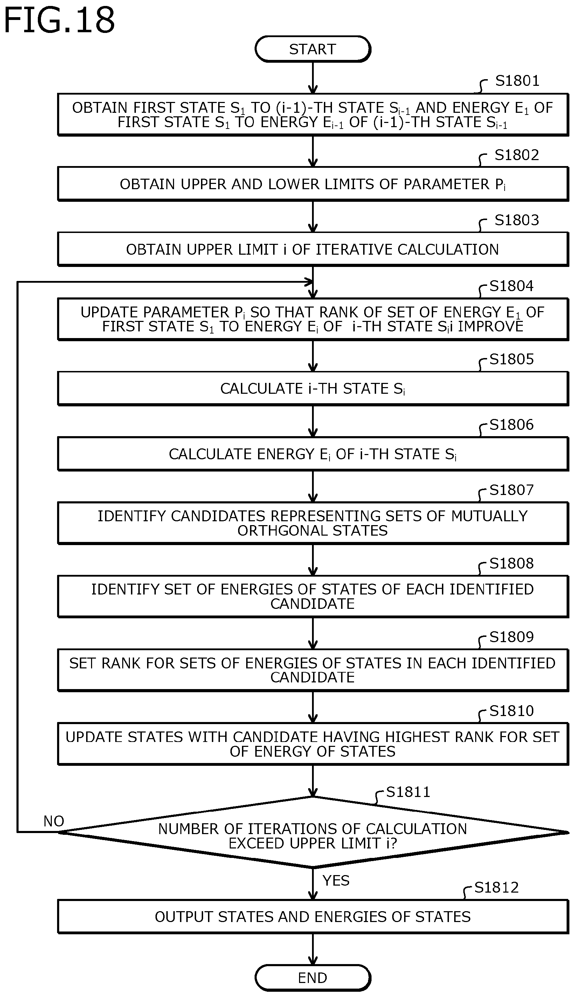

Next, with reference to Fig. 18, an example of the i-th energy calculation processing procedure executed by the excitation energy calculating device 201 is described. The i-th energy calculation processing is realized, for example, by the CPU 301, storage areas such as the memory 302 and the recording medium 305, and the network I/F 303 depicted in Fig. 3.

Fig. 18 is a flowchart depicting an example of the i-th energy calculation processing procedure. In Fig. 18, the excitation energy calculating device 201 obtains each state from the first state S₁ to the (i-1)-th state Sᵢ₋₁ and each energy thereof. The excitation energy calculating device 201 obtains each state from the first state S₁ to the (i-1)-th state Sᵢ₋₁ and each energy from the energy E₁ of the first state S₁ to the energy Eᵢ₋₁ of the (i-1)-th state Sᵢ₋₁ (step S1801).

The excitation energy calculating device 201 obtains the upper and lower limits of the parameter Pᵢ representing the i-th state Sᵢ (step S1802). The excitation energy calculating device 201 obtains the upper limit i of the number of iterations of calculation (step S1803). The excitation energy calculating device 201 proceeds to the process at step S1804.

At step S1804, the excitation energy calculating device 201 updates the parameter Pᵢ such that the ranking improves for each energy set from the energy E₁ of the first state S₁ to the energy Eᵢ of the i-th state Sᵢ (step S1804).

The excitation energy calculating device 201 calculates the i-th state Sᵢ based on the parameter Pᵢ (step S1805). The excitation energy calculating device 201 calculates the energy Eᵢ of the i-th state Sᵢ based on the calculated i-th state Sᵢ (step S1806). The excitation energy calculating device 201 identifies candidates representing sets including mutually orthogonal states by fixing each state and correcting other states so that the states are orthogonal to each other (step S1807).

The excitation energy calculating device 201 identifies a set of energies for each state in each identified candidate (step S1808). The excitation energy calculating device 201 sets a rank for each set of energies of states in the identified candidates (step S1809). The excitation energy calculating device 201 updates each state using the candidate with the highest rank for each set of energies of states (step S1810).

The excitation energy calculating device 201 determines whether the number of iterations of calculation has exceeded the upper limit i (step S1811). Here, when the number of iterations of calculation has not exceeded the upper limit i (step S1811: NO), the excitation energy calculating device 201 returns to the process at step S1804. On the other hand, when the number of iterations of calculation has exceeded the upper limit i (step S1811: YES), the excitation energy calculating device 201 proceeds to the process at step S1812.

At step S1812, the excitation energy calculating device 201 outputs each last-updated state and the energy of each last-updated state (step S1812). The excitation energy calculating device 201 may also output each parameter, each last-updated state, and the energy of each last-updated state. The excitation energy calculating device 201 completes the i-th energy calculation process. This enables the excitation energy calculating device 201 to calculate the i-th state Sᵢ and the energy Eᵢ of the i-th state Sᵢ.

As described above, according to the information processing device 100, the excitation state to be calculated can be calculated based on the parameter values. According to the information processing device 100, based on the calculated excitation state to be calculated, candidates representing sets of mutually orthogonal states can be generated. According to the information processing device 100, a set of energies for each state in each generated candidate can be calculated. According to the information processing device 100, it is possible to set the rank for each set of calculated state energies in each candidate such that a point corresponding to a set and relatively closer to the origin in the coordinate system has a relatively higher rank. According to the information processing device 100, it is possible to update each state to one of the candidates based on the set ranks. According to the information processing device 100, the values of the parameters can be updated such that the rank for the set of energies of each updated state becomes higher in the coordinate system. This enables the information processing device 100 to accurately calculate the excited state that is to be calculated while satisfying the constraint that the states are orthogonal, whereby accurate calculation of the energy of the excited state to be calculated becomes possible.

According to the information processing device 100, the ground state can be obtained by repeating a computation for calculating the ground state, based on the parameter values through quantum chemical calculations while changing the parameter values representing the ground state. This enables the information processing device 100 to accurately calculate the ground state and make the calculated ground state available for reference when calculating the energy of the excited state that is to be calculated.

According to the information processing device 100, a first excited state with the next lowest energy after the ground state can be calculated based on the values of the first parameter. According to the information processing device 100, candidates representing sets of mutually orthogonal states can be generated based on the ground state and the calculated first excited state. According to the information processing device 100, the set of energies for each state in each generated candidate can be calculated. According to the information processing device 100, it is possible to set a rank for the calculated set of energies of each state in each candidate such that a point corresponding to a set and relatively closer to the origin in the first coordinate system has a relatively higher rank. According to the information processing device 100, it is possible to update each state to one of the candidates based on the set rank. According to the information processing device 100, the value of the first parameter can be changed such that the rank for the set of energies of each updated state becomes higher in the first coordinate system. This enables the information processing device 100 to accurately calculate the first excited state, which has the next lowest energy after the ground state, and to accurately calculate the energy of the first excited state.

According to the information processing device 100, based on the values of the second parameter, it is possible to calculate the next lowest energy excited state that is to be calculated following the previously calculated excited state. According to the information processing device 100, based on the ground state and the calculated excited state to be calculated, it is possible to generate candidates representing sets of mutually orthogonal states. According to the information processing device 100, it is possible to calculate a set of energies for each state in each generated candidate. According to the information processing device 100, it is possible to set the rank for the set of energies calculated for each state in each candidate such that a point corresponding to a set of energies calculated for each state and relatively closer to the origin in the second coordinate system has a higher rank. According to the information processing device 100, it is possible to update each state to one of the candidates based on the set rank. According to the information processing device 100, the values of the second parameter can be changed such that the rank for the set of energies of the updated state becomes higher in the second coordinate system. This enables the information processing device 100 to accurately calculate the excited state that is to be calculated and to accurately calculate the energy of the target excited state.

According to the information processing device 100, after repeating a specific computation, the energy of each state last updated can be output. This allows the information processing device 100 to make the energy of each state available for external use.

According to the information processing device 100, in a coordinate system, for any of the energies of the calculated states, when another set of calculated energies for states is present having the same distance from the origin and the energy of the ground state is lower than that of the other set, a rank higher than that of the other set may be set. This allows the information processing device 100 to appropriately evaluate the sets of energies of states.

According to the information processing device 100, each state can be updated to one of the candidates with the highest set rank. This allows the information processing device 100 to appropriately update each state.

According to the information processing device 100, using an optimization algorithm, for each updated state, the parameter values can be changed by searching within a preset range for values that increase the rank relative to the next calculated energy set. This allows the information processing device 100 to change the parameter values in a desirable direction.

The information processing method described in the present embodiment may be implemented by executing a prepared program on a computer such as a personal computer and a workstation. The program is stored on a computer-readable recording medium such as a hard disk, a flexible disk, a compact disc read-only memory (CD-ROM), a magnetooptical (MO) disc, and a digital versatile disc (DVD), read out from the computer-readable medium, and executed by the computer. The program may be distributed through a network such as the Internet.

### EXPLANATIONS OF LETTERS OR NUMERALS

100 information processing device
200 information processing system
201 excitation energy calculating device
202 client device
210 network
300 bus
301 CPU
302 memory
303 network interface
304 recording medium interface
305 recording medium
400 energy calculation settings
401 energy count
402 parameter range
403 upper limit of number of iterations of calculation
500 controller
501 obtaining unit
502 first optimization calculating unit
503 second optimization calculating unit
504 output unit
510 storage unit
601 first parameter calculating unit
602 first state calculating unit
603 first energy calculating unit
604 first determining unit
701 second parameter calculating unit
702 second state calculating unit
703 second energy calculating unit
704 second rank calculating unit
705 second determining unit
800 first calculation result table
801, 802, 803, 804 vector
900 second calculation result table
1100 coordinate system
1101, 1102, 1103, 1104, 1105, 1106, 1107 point
1300 output result table

## Claims

1. An information processing program for causing a computer to execute a process, the process comprising:
when repeating by quantum chemical calculation, a specific computation for calculating each of a plurality of potential excited states of a substance, the specific computation being repeated based on a value of a parameter while changing the value of the parameter that is for expressing a target excited state to be calculated among the plurality of excited states,
calculating based on the value of the parameter, the target excited state to be calculated;
based on the calculated target excited state, for each of the plurality of potential excited states of the substance from a ground state of the substance to the target excited state to be calculated, fixing the each of the plurality of potential excited states of the substance and subtracting a projection component onto at least one state from another state so that the states are orthogonal to each other, thereby generating a plurality of candidates each representing a set of the states orthogonal to each other;
calculating a set of energies of the states in each of the generated plurality of candidates;
setting a rank for each calculated set of energies so that in a coordinate system containing axes representing the energies of each state, among a plurality of points respectively corresponding to the calculated sets of energies, a point relatively closer to an origin has a relatively higher rank;
updating each state with one of the plurality of candidates, based on the set rank; and
changing the value of the parameter so that in the coordinate system, the rank set for each set of energies of each updated state increases.

2. The information processing program according to claim 1, the process further comprising obtaining the ground state by repeatedly calculating by the quantum chemical calculation, the ground state based on a value of a parameter representing the ground state, while changing the value of the parameter representing the ground state.

3. The information processing program according to claim 2, the process further comprising:
when repeating by the quantum chemical calculation, a first computation for calculating a first excited state based on a value of a first parameter while changing the value of the first parameter that is for expressing the first excited state having a next lowest energy from the ground state,
calculating the first excited state based on the value of the first parameter;
based on the ground state and the calculated first excited state, for each of the plurality of potential excited states of the substance from the ground state to the first excited state, fixing the each of the plurality of potential excited states of the substance and subtracting a projection component onto at least one state from another state so that the states are orthogonal to each other, thereby generating a plurality of candidates each representing a set of the states orthogonal to each other;
calculating a set of energies of the states in each of the generated plurality of candidates;
setting a rank for each calculated set of energies so that in a first coordinate system having axes representing the energies of each state, among a plurality of points respectively corresponding to the calculated sets of energies, a point relatively closer to the origin has a relatively higher rank;
updating each state with one of the plurality of candidates, based on the set rank; and
changing the value of the first parameter so that in the first coordinate system, the rank set for each set of energies of each updated state increases.

4. The information processing program according to claim 3, the process further comprising:
when repeating by the quantum chemical calculation, a second computation for calculating the target excited state based on a value of a second parameter while changing the value of the second parameter that is for expressing the target excited state to be calculated having a next lowest energy after that the calculated excited state,
calculating the target excited state based on the value of the second parameter;
based on the ground state and the calculated target excited state, for each of the plurality of potential excited states of the substance from the ground state of the substance to the target excited state to be calculated, fixing the each of the plurality of potential excited states of the substance and subtracting a projection component onto at least one state from another state so that the states are orthogonal to each other, thereby generating a plurality of candidates each representing a set of the states orthogonal to each other;
calculating a set of energies of the states in each of the generated plurality of candidates;
setting a rank for each calculated set of energies so that in a second coordinate system having axes representing the energies of each state, among a plurality of points respectively corresponding to the calculated sets of energies, a point relatively closer to the origin has a relatively higher rank;
updating each state with one of the plurality of candidates, based on the set rank; and
changing the value of the second parameter so that in the second coordinate system, the rank for each set of energies of each updated state increases.

5. The information processing program according to claim 1, the process further comprising:
outputting each set of energies of each state last updated after repeating the specific computation.

6. The information processing program according to claim 1, wherein for any calculated set of energies, when another calculated set of energies a same distance from the origin is present in the coordinate system and an energy of the ground state is lower than that of the other calculated set of energies, the setting includes setting a higher rank than that for the other calculated set of energies, and when the energy of the ground state is higher than that of the other calculated set of energies, the setting includes setting a lower rank than that for the other calculated set of energies.

7. The information processing program according to claim 1, wherein the updating includes:
updating each state with one of the plurality of candidates having a highest rank.

8. The information processing program according to claim 1, wherein
the changing includes using an optimization algorithm and for each of the updated states, changing the value of the parameter by searching, within a predetermined range, for a value of the parameter that increases the rank for a next set of energies to be calculated.

9. An information processing method executed by a computer, the method comprising:
when repeating by quantum chemical calculation, a specific computation for calculating each of a plurality of potential excited states of a substance, the specific computation being repeated based on a value of a parameter while changing the value of the parameter that is for expressing a target excited state to be calculated among the plurality of excited states,
calculating based on the value of the parameter, the target excited state to be calculated;
based on the calculated target excited state, for each of the plurality of potential excited states of the substance from a ground state of the substance to the target excited state to be calculated, fixing the each of the plurality of potential excited states of the substance and subtracting a projection component onto at least one state from another state so that the states are orthogonal to each other, thereby generating a plurality of candidates each representing a set of the states orthogonal to each other;
calculating a set of energies of the states in each of the generated plurality of candidates;
setting a rank for each calculated set of energies so that in a coordinate system containing axes representing the energies of each state, among a plurality of points respectively corresponding to the calculated sets of energies, a point relatively closer to an origin has a relatively higher rank;
updating each state with one of the plurality of candidates, based on the set rank; and
changing the value of the parameter so that in the coordinate system, the rank set for each set of energies of each updated state increases.

10. An information processing device comprising a controller, the controller being configured to:
when repeating by quantum chemical calculation, a specific computation for calculating each of a plurality of potential excited states of a substance, the specific computation being repeated based on a value of a parameter while changing the value of the parameter that is for expressing a target excited state to be calculated among the plurality of excited states,
calculating based on the value of the parameter, the target excited state to be calculated;
based on the calculated target excited state, for each of the plurality of potential excited states of the substance from a ground state of the substance to the target excited state to be calculated, fixing the each of the plurality of potential excited states of the substance and subtracting a projection component onto at least one state from another state so that the states are orthogonal to each other, thereby generating a plurality of candidates each representing a set of the states orthogonal to each other;
calculating a set of energies of the states in each of the generated plurality of candidates;
setting a rank for each calculated set of energies so that in a coordinate system containing axes representing the energies of each state, among a plurality of points respectively corresponding to the calculated sets of energies, a point relatively closer to an origin has a relatively higher rank;
updating each state with one of the plurality of candidates, based on the set rank; and
changing the value of the parameter so that in the coordinate system, the rank set for each set of energies of each updated state increases.
